(19) Europäisches Patentamt
European Patent Office
Office européen des brevets

(11) EP 0 528 034 B1

(12) **EUROPEAN PATENT SPECIFICATION**

(45) Date of publication and mention of the grant of the patent:
**21.04.1999 Bulletin 1999/16**

(21) Application number: **91909364.1**

(22) Date of filing: **17.05.1991**

(51) Int. Cl.$^{6}$: **A61K 38/00**, A61K 35/42, A61K 31/685

(86) International application number:
**PCT/JP91/00664**

(87) International publication number:
**WO 91/17766 (28.11.1991 Gazette 1991/27)**

(54) **REMEDY FOR ASTHMA**

ASTHMA HEILMITTEL

REMEDE CONTRE L'ASTHME

(84) Designated Contracting States:
**AT BE CH DE DK ES FR GB GR IT LI LU NL SE**

(30) Priority: **18.05.1990 JP 126936/90**

(43) Date of publication of application:
**24.02.1993 Bulletin 1993/08**

(73) Proprietor:
**TOKYO TANABE COMPANY LIMITED**
**Tokyo 103-8405 (JP)**

(72) Inventor:
**KURASHIMA, Kazuyoshi,**
**20-21, Asahi-machi 2-chome**
**Ishikawa 920 (JP)**

(74) Representative:
**Calamita, Roberto**
**Frank B. Dehn & Co.,**
**European Patent Attorneys,**
**179 Queen Victoria Street**
**London EC4V 4EL (GB)**

(56) References cited:
**EP-A- 0 110 498**
**EP-A- 0 119 056**
**JP-A- 1 063 526**
**JP-A-58 164 513**
**JP-A-58 183 621**
**JP-A-60 237 023**
**JP-A-61 065 821**
**US-A- 4 397 839**

- **Rudolf Voigt, LEHRBUCH DER PHARMAZEUTISCHEN TECHNOLOGIE, 5. Auflage, Verlag Chemie , 1984**
- **JAPANESE JOURNAL OF ALLERGOLOGY vol. 40, no. 2, February 1991, pages 160 - 163 K. KURASHIMA ET AL. 'A PILOT STUDY OF SURFACTANT INHALATION FOR THE TREATMENT OF ASTHMATIC ATTACK'**
- **BIOMEDICA BIOCHIMICA ACTA vol. 44, no. 9, 1985, pages K57 - K61 G. BECHER 'LUNG SURFACTANT PREVENTS ALLERGIC BRONCHIAL CONSTRICTION IN OVALBUMIN SENSITIZED GUINEA PIGS'**
- **PEDIATRICS vol. 71, no. 6, 1983, pages 913 - 917 J.A. SMYTH ET AL. 'HYALINE MEMBRANE DISEASE TREATED WITH BOVINE SURFACTANT'**
- **LIPIDS vol. 18, no. 8, 1983, pages 522 - 529 S. YU ET AL. 'BOVINE PULMONARY SURFACTANT'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 528 034 B1

## Description

This invention relates to the use of a pulmonary surface active material (hereinafter abridged as PSF) in the manufacture of a medicament for the treatment of asthma in humans.

BACKGROUND ART

It is generally realized that bronchial asthma is a very complex syndrome with various causes and its dominating clinical symptoms are not constant.

On the other hand, PSF is specially evaluated as a therapeutic agent for respiratory distress syndrome which has a high mortality. The main function of PSF is to reduce surface tension (i.e. free energy of the surface per unit area) of the air-liquid interface and thereby provides mechanical stability to alveolar units. PSF exists not only in alveoli but also in airways and stabilizes bronchi, thereby protectig bronchi against radius changes with lung volume. Further, PSF contributes importantly to pulmonary function, including reduction of breathing work and control of fluid transudation which protects agains development of pulmonary edema. In fact, in edema induced artificially by increasing alveolar surface tension, an exogenous PSF can be administered to reduce protein permeability in alveoli of premature lambs. In asthmatic attack, mucus secretion increases and clearance of mucosal cilia is disturbed. Increases of mucus secretion and transudation of proteinous fluid may inhibit surfactant activity in small airways and alveoli. Increased surface tension and transudation causes occlusion by the fluid which transuded during exhalation in the airways closest to the alveoli. It is considered that if surface activity has something to do with the peripheral airways in asthmatic attack, administration of PSF have some therapeutic effects in asthmatic attack. In practice, however, the efficacy of PSF as an antasthmatic has not been known yet.

Baker G: Biomed. Biochem. Acta, Vol. 44, 1985, pages K57-K61 discloses the use of natural surfactant to reduce bronchial obstruction after intratracheal ovalbumin challenge in artificially ventilated guinea-pigs sensitised to ovalbumin, and the use of such natural surfactant in protecting against receptor mediated allergic reaction in the lungs and bronchi.

The inventor has eagerly researched for a drug useful as an antasthmatic and found that PSF has marked therapeutic effects in guinea pigs with antigen-induced broncho-constriction as a model of bronchial asthma and, in fact, in patients with asthma, leading to the present invention.

DISCLOSURE OF THE INVENTION

This invention provides the use of a PSF as an effective ingredient in the preparation of a medicament for the treatment of asthma in humans.

As PSF in the antasthmatic of the present invention, can be used various known materials containing phospholipid by 40 w/w% or more as a whole, including phosphatidylcholine or choline phosphoglyceride as a main component. In detail, as PSF used in the present invention, can be listed (i) a surface active material which contains phospholipids, neutral lipids, total cholesterol, carbohydrates and proteins, of origin of mammarian pulmonary tissue, respectively at 75.0-95.5 w/w%, 1.8-14.0 w/w%, 3.0 w/w% or less, 0.1-1.5 w/w%, and 5.0 w/w% or less of total weight of the dried final product (Japanese Patent Publication No. 61-9925); (ii) a pulmonary surface active pharmaceutical composition mainly consisting of dipalmitoylphosphatidyl choline and fatty alcohols (Japanese Unexamined Patent Laid-open No. 57-99524); (iii) a surface active material (referred to as Surfactant TA in the following) containing phospholipids, neutral lipids, total cholesterol, free fatty acids, carbohydrates and proteins, of origin of mammalian pulmonary tissue, respectively at 68.6-90.7 w/w%, 0.3-13.0 w/w%, 0.0-8.0 w/w%, 1.0-27.7 w/w%, 0.1-2.0 w/w% and 0.0-3.5 w/w% of the total dry weight (Japanese Patent Publication No. 61-9924); (iv) a synthetic surface active material mainly containing phospholipid phosphatidyl choline and unsaturated fatty acids or their esters, the proportion of the phosphatidyl choline being 55-80 w/w% of the total (Japanese Unexamined Patent Laid-open No. 58-135813); (v) a pulmonary surface active material containing phospholipids at 80 w/w% or more of the total and containing substantially no proteins (Japanese Unexamined Patent Laid-open No. 58-164513); (vi) a pulmonary surface active material containing phospholipids, neutral lipids, cholesterols and carbohydrates, extracted from mammalian lung, in the component ratios of 70-95 w/w%, 1-10 w/w%, 3.0 w/w% or less, and 0.3 w/w% or less, respectively, after drying, and containing substantially no proteins (Japanese Patent Unexamined Laid-opn No. 58-183620); (vii) a synthetic pulmonary surface active material containing phospholipid phosphatidyl choline and cardiolipin as main ingredients, the ratio of the phosphatidyl choline being 55-80 w/w% of the total (Japanese Patent Publication No. 1-29171); (viii) a pulmonary surfactant containing 40-45 w/w% of dipalmitoylphosphatidyl choline, 5-10 w/w% of dipalmitoylphosphatidylglycerin and 50 w/w% of sugars (Japanese Patent Publication No. 1-13690); (ix) a synthetic pulmonary surface active material (referred to as Surfactant CL in the following) containing 80-95 w/w% of phosphatidyl choline, cardiolipin and/or phosphatidyl glycerol, as phospholipids, 5-20 w/w% of neutral lipids and 0-10 w/w% of fatty acids (Japanese Unexamined Patent Laid-open No. 59-95219, Journal of Japan Surface Medicine Society, Vol.14, No.1: p.59, 1983); (x) a surfactant (referred to as Synthetic Surfactant X1 in the following) mainly containing choline phosphoglyceride, acid phospholipids, fatty acids, and mammalian lung-derived lipoproteins, respectively at 50.6-85.0 w/w%, 4.5-37.6 w/w%, 4.6-24.6 w/w% and 0.1-10.0 w/w% (Japanese Unexamined Patent Laid-open No. 59-164724); (xi) a synthetic pulmonary surface active material containing 55-80 w/w% of phosphatidyl choline having 2 residues of saturated straight chain fatty acid, 10-35 w/w% of phosphatidyl glycerol having 2 residues of saturated straight chain fatty acid and 5-20 w/w% of neutral lipids (Japanese Unexamined Patent Laid-open No. 59-181216); (xii) a mixture of agonists containing 40-70 % of phospholipds, less than 1.5 % of proteins, 10-40 % of cholesterols and 5-30 % of neutral lipids (Japanese Unexamined Patent Laid-open No. 60-237023); (xiii) a synthetic surfactant (referred to as Synthetic Surfactant X2 in the following) mainly containing choline phosphoglyceride, acid phospholipids and fatty acids respectively at 53.9-87.8 w/w%, 4.8-38.2 w/w% and 7.0-26.2 w/w% of the total weight (Japanese Patent Publication No. 2-8768); (xiv) a material (referred to as Surfactant CK in the following) consisting of lipids extracted from porcine alveolar washing solution to which calcium chloride is added (Journal of Japan Surface Medicine Society, Vol.12, No.1, p.1, 1981 and Vol.14, No.2, p.212, 1983); (xv) a synthetic pulmonary surface active material containing a mixture of 3 components system including dipalmitoyl phosphatidyl choline, distearoyl phosphadyl choline and soybean lecithin (Japanese Patent Publication No. 64-9292); (xvi) a pulmonary arterial surfactant originating from animal comprising a polar lipid fraction and a protein fraction, which is composed of at least 98.5 w/w% of the polar lipids, and mainly composed of at least 95 % of a mixture of phospholipids (Japanese Unexamined Patent Laid-open No. 64-63526); (xvii) a synthetic pulmonary surface active material containing the pulmonary surface active material proteins described in and manufactured with the pulmonary surface active material proteins described in Japanese Kohyo No. 62-501122, Japanese Kohyo No. 62-501792, Japanese Kohyo No. 63-503222, Japanese Kohyo No. 1-501282, Japanese Unexamined Patent Laid-open No. 2-424, Japanese Unexamined Patent Laid-open No. 2-6405, Japanese Unexamined Patent Laid-open No. 2-53798, Japanese Unexamined Patent Laid-open 2-279628, Japanese Kohyo No. 2-502917, Japanese Unexamined Patent Laid-open No. 3-44332 and Japanese Unexamined Patent Laid-open No. 3-90033, or, in addition, pulmonary surface active material proteins produced by gene recombination techniques; and (xviii) a natural PSF or a product prepared from it, such as Alveofact (tradename, See Eur. J. Pediatr. (1990) 149: 280-283; and LIPIDS, Vol.18, No.8 (1983) 522-529) obtained from bovine alveoli and comprising phospholipids, cholesterols, hydrophobic surface active proteins, free fatty acids, triglycerides and calcium; Infasurf (tradename); Curosurf (tradename); and Humansurf (tradename) obtained from human waters; (xix) a synthetic PSF such as Exosurf (tradename) comprising Surfactant CK, dipalmitoil phosphatidyl choline, hexadecanol, Tyloxapol (formaldehyde polymer with oxirane and 4-(1,1,3,3-tetramethylbutyl)phenol) and sodium chloride; ALEC (tradename) comprising 7 parts of dipalmitoyl phosphatidyl choline and 3 parts of phosphatidyl glycerol; Dry Surfactant; and Liposomalform.

Also, a dispersing concentration of PSF in a range of 0.1-100.0 mg/ml, preferably 1-50 mg/ml, and more preferably 2-40 mg/ml, is suitable.

BRIEF DESCRIPTION OF DRAWINGS

Fig. 1 is a schematic view of an apparatus used as a body plethysmograph for a guinea pig;
Fig. 2 is a graph explaining calculations of Cdyn and RL;
Fig. 3 is a graph relating to Surfactant TA, showing percent changes in pressure at airway opening (Pao) from the baseline after antigen challenge in three groups of experimental animals;
Fig. 4 is a graph relating to Surfactant TA, showing percent changes in dynamic compliance of lung (Cdyn) from the baseline;
Fig. 5 is a graph relating to Surfactant TA, showing percent changes in pulmonary resistance (RL) from the baseline;
Fig. 6 is a graph showing effects of inhalation of Surfactant TA for 90 seconds against bronchoconstriction induced by histamine;
Fig. 7 is a graph relating to ALEC, showing percent changes in pressure at airway opening (Pao) after antigen challenge in three groups of experimental animals;
Fig. 8 is a graph relating to ALEC, showing percent changes in dynamic compliance of lung (Cdyn) from the baseline;
Fig. 9 is a graph relating to ALEC, showing percent changes in pulmonary resistance (RL) from the baseline;
Fig. 10 is a graph relating to Exosurf, showing percent changes in pressure at airway opening (Pao) after antigen challenge in three groups of experimental animals;
Fig. 11 is a graph relating to Exosurf, showing percent changes in dynamic compliance of lung (Cdyn) from the baseline;

Fig. 12 is a graph relating to Exosurf, showing percent changes in pulmonary resistance (RL) from the baseline;

Fig. 13 is a graph relating to Alveofact, showing percent changes in pressure at airway opening (Pao) after antigen challenge in three groups of experimental animals;

Fig. 14 is a graph relating to Alveofact, showing percent changes in dynamic compliance of lung (Cdyn) from the baseline;

Fig. 15 is a graph relating to Alveofact, showing percent changes in pulmonary resistance (RL) from the baseline;

Fig. 16 is a graph relating to Surfactant CK, showing percent changes in pressure at airway opening (Pao) after antigen challenge in three groups of experimental animals;

Fig. 17 is a graph relating to Surfactant CK, showing percent changes in dynamic compliance of lung (Cdyn) from the baseline;

Fig. 18 is a graph relating to Surfactant CK, showing percent changes in pulmonary resistance (RL) from the baseline;

Fig. 19 is a graph relating to Humansurf, showing percent changes in pressure at airway opening (Pao) after antigen challenge in three groups of experimental animals;

Fig. 20 is a graph relating to Humansurf, showing percent changes in dynamic compliance of lung (Cdyn) from the baseline; and

Fig. 21 is a graph relating to Humansurf, showing percent changes in pulmonary resistance (RL) from the baseline.

[Effects on a bronchial experimental model]

Preparation of asthma model

Preparation of high titer anti-ovalbumin antiserum

Guinea pig homocytotropic antiserum was made by the modified method of Santives et al (Santives T, Roska AK, Henly G, Moore VL, Fink JN, Abramoff P: Immunologically induced lung disease in guinea pig; J Allergy Clin Immunol 1976; 57: 582-594). A dose of 500 μg ovalbumin (OA) was emulsified in complete Freund's adjuvant and administered intradermally into each guinea pig at 5 different areas, i.e., bilateral axilla, bilateral inguinal region and neck. Boostering was carried out in the same manner 2 weeks later. The serum was collected 2 weeks after the booster, pooled and kept frozen until use.

Induction of experimental asthma

Guinea pigs were passively sensitized with 1 ml/kg of the above-described antiserum administered intraperitoneally. Twelve to 24 h after the passive sensitization, the guinea pigs were anesthetized with an intraperitoneal injection of 75 mg/kg pentobarbital sodium. They were placed in the supine position, the trachea was cannulated with a polyethylene tube (outer diameter 2.5 mm; inner diameter 2.1 mm), and one jugular vein was cannulated for the administration of drugs. The animals were artificially ventilated by a small animal ventilator (Model 1680, Harvard Apparatus, South Natick, MA) adjusted to a tidal volume of 10 ml/kg at a rate of 60 breaths/min. Mechanical dead space in this system was 0.5 ml. When all the above procedures were completed, the animals were given 60 mg/kg diphenhydramine hydrochloride intraperitoneally to block the action of histamine completely, and overinflated by 2 times of tidal volume by clamping the outlet port of the respirator. Ten minutes later, the animals were challenged with nebulized ovalbumin (antigen) dissolved in saline (1 mg/ml) without interrupting the artificial ventilation. The ovalbumin aerosol was generated for 30 seconds by an apparatus equiped with an ultrasonic nebulizer, developed for small animal experiments.

Pulmonary suface active material

Surfactant TA (Surfacten, Tokyo Tanabe Company, Ltd., Tokyo; 120 mg lyophilized pulmonary surfactant lipid in a vial) was suspended in warmed (37°C) saline to give a lipid concentration of 10 and 20 mg/ml.

Other drugs used were as follows: ovalbumin (Sigma, St. Louis, MO), complete Freund's adjuvant (Difco Laboratories, Detroit, MI), diphenhydramine HCl (Sigma, St. Louis, MO), pentobarbital sodium solution (Abbott Laboratories, North Chicago, IL), histamine dihydrochloride (Wako pure chemical industries, Osaka, Japan).

Measurements

As shown in Fig. 1, pressure at airway opening (Pao), lateral pressure of tracheal tube, was measured using a differential pressure transducer (MOdel TP-603T, Nihon Koden, Tokyo Japan). In addition, esophageal pressure (Peso) was measured by a water-filled polyethylene catheter (outer diameter 1.4 mm; inner diameter 1.0 mm) which was inserted into the esophagus and connected to a low-pressure transducer (Model MPU-0.1A, Nihon Koden). Thereafter, the guinea pigs were kept in a small airtight plastic box, and a flow rate ($\dot{V}$) of the air was measured with a Lilly-type pneumatograph (Model TV241T, Nihon Koden) and a low-pressure transducer (Model TP-602T, Nihon Koden) placed at the small window of the box. The flow rate was electronically integrated and the ventilation volume (V) was measured. These parameters, $\dot{V}$, V, Pao and Peso, were recorded continuously on a multi-channel recorder (Model P-0770c, Nihon Koden). According to the methods described by Amdur Mo, and Mead J: Mechanics of respiration in unanesthetized guinea pigs; Am J Physiol 1958; 192: 346-368, dynamic compliance of lung (Cdyn) and pulmonary resistance (RL) were calculated by the equations shown in Fig. 2.

Cdyn was divided by body weight (kg), and RL used was the value multiplied by the body weight. In order to compare the changes in peripheral airway and those in primary bronchus, the ratio of Cdyn and reciprocal RL (1/RL) to the baseline were calculated.

Pao was measured continuously, and Cdyn and RL were measured shortly before the antigen inhalation (base line), at its peak, 18 and 23 minutes after the antigen challenge. The changes in these values at each time were expressed as percent changes of the baseline value, i.e., %Pao, %Cdyn, %1/RL, respectively. Recovery rate was determined as percent change of the value at 23 minutes to that at 18 minutes.

Protocol

A total of 27 guinea pigs were studied according to the following protocol. After the administration of diphenhydramine hydrochloride, measurements of respiratory function were performed (baseline). Then, the animals were challenged with nebulized ovalbumin. About 14 minutes later when the Pao reached its maximum value, second measurments of respiratory function were performed. At 18 minutes later when the bronchoconstriction became sustained phase where the rate of decrease in Pao became slow, third measurements of respiratory function were performed (before administering PSF). Twenty minutes later, either Surfactant TA 10 mg/ml (a PSF 10 mg/ml treated group, n=9), 20 mg/ml (a PSF 20 mg/ml treated group, n=9), or saline (a control group, n=9) was inhaled for 90 minutes. Inhalants were given by the ultrasonic nebulizer described above. The median aerodynamic diameters of the particles of saline, PSF 10 mg/ml, PSF 20 mg/ml produced by the nebulizer were 3.59 ± 1.96 μm (mean ± SD), 3.64 ± 1.87 μm, 3.63 ± 1.98 μm, respectively. Fourth measurements of respiratory function were performed 23 minutes after ovalbumin challenge (1.5 minutes after the end of PSF or saline inhalation).

Histamine-induced bronchoconstriction

Other guinea pigs (n=10) were anesthetized and artificially ventilated as described above, and then, ascending doses of histamine were intravenously administered at 5 minute intervals without interrupting the ventilation. In 5 guinea pigs, inhalant of Surfactant TA 20 mg/ml was inhaled for 90 seconds, 10 minutes before the histamine provocation. As the control, inhalant of saline was given to the other 5 guinea pigs in the same manner.

Statistical Analysis

Data are presented as mean ± SEM, and the statistical differences were determined by Mann-Whitney's U test, taking a P value of 0.05 or less as significant.

Results

Effects of PSF inhalation in the asthmatic model

Baseline values of Pao, Cdyn, RL in all the experimental animals were 10.5 ± 0.27 $cmH_2O$, 1.33 ± 0.07 $ml/cmH_2O \cdot kg$, 6.91 ± 0.27 $cmH_2O \cdot sec \cdot kg10^{-2}/ml$, respectively. There were no significant differences in these values among the groups.

As shown in Fig. 3, percent increase of Pao reached its maximum value of 267 ± 39 % at 13 minutes after the inhalation in the control group (n=9), which was followed by decrease to 245 ± 32 % at 18 minutes, and to 222 ± 25 % at 23 minutes. In the group treated with 10 mg/ml of PSF, percent increase of Pao reached its maximum value of 269 ± 23 % in 15 minutes, and decreased to 253 ± 23 % at 18 minutes, and to 210 ± 22 % at 23 minutes. In the group treated with 20 mg/ml of PSF, percent increase of Pao reached the maximum value of 272 ± 32 % in 14 minutes, and thereafter, decreased to 252 ± 29 % at 18 minutes, and to 196 ± 31 % at 23 minutes. Recovery rate of Pao (percent change of the value at 23 minutes from that at 18 minutes) was 5.9 ± 2.4 % in the control group, 20.7 ± 2.2 % ($p < 0.01$) in the group treated with 10 mg/ml of PSF, and 23.4 ± 4.7 % ($p < 0.01$) in the group treated with 20 mg/ml of PSF.

Fig. 4 shows changes of % Cdyn in the present experiment. The minimum value of % Cdyn of all the guinea pigs was 10.3 ± 0.3 %, which was seen at 14.3 ± 0.5 minutes after the antigen challenge. There was no significant difference in the minimum value and its appearance time among the three groups. On the other hand, recovery rates of Cdyn were 14.6 ± 1.9 % in the control group, 43.5 ± 10.3 % ($p < 0.02$) in the group treated with 10 mg/ml of PSF, and 52.0 ± 9.5 % ($p < 0.01$) in the group treated with 20 mg/ml of PSF. Therefore, Cdyn recovered more rapidly in PSF-treated groups.

Changes in % 1/RL are shown in Fig. 5. The minimum value of % 1/RL of all the experimental animals was 13.7 ± 0.9 %. There was no significant difference in the minimum value among these groups. Recovery rate of 1/RL was 34.5 ± 15.7 % in the control group, 39.5 ± 5.5 % (NS) in the group treated with 10 mg/ml of PSF, and 102.4 ± 15.7 % ($p < 0.01$) in the group treated with 20 mg/ml of PSF. Therefore, with regard to the change of 1/RL, there was no significant difference between the control group and the group treated with 10 mg/ml of PSF, but the recovery rate in the group treated with 20 mg/ml of PSF was significantly greater than that in the other groups.

Influence of PSF inhalation on histamine-induced bronchoconstriction

Fig. 6 shows the influence of inhaling 20 mg/ml of PSF for 90 seconds on histamine-induced bronchoconstriction. No direct bronchodilative effect of PSF inhalation was observed in this experiment.

Discussion

The guinea pigs used in this experiment constitute one of well characterized experimental models for bronchial asthma. The guinea pigs were passively sensitized with homocytotropic antiserum and pretreated with a high dose of diphenhydramine hydrochloride to block the bronchoconstriction mediated by endogenous histamine completely. By blocking the effect of histamine, dose-response and reproducibility are available, and hypersensitivity reaction occurs when the animals are challenged with an aerosol antigen. In the previous study of the inventor, this type of allergic bronchoconstriction was inhibited for the most part by continuous infusion of FPL55712, a selective inhibitor for the slow reacting substance of anaphylaxis (SRS-A), or by the pretreatment with inhalation of AS-35, a leukotriene receptor antagonist. From these facts, the bronchoconstriction following ovalbumine inhalation seen in the present experiment should be an allergic one mediated mainly by SRS-A.

A problem in the aerosolization of PSF is that only a limited quantity of the liquid can be deposited within lungs. Based on the data of Oyarzun MJ and Clements JA: Control of lung surfactant by ventilation, adrenergic mediators, and prostaglandins in the rabbit; Am Rev Respir Dis 1987; 117: 879-91, on total phospholipids recovered by lung lavage of rabbits (2.5 mg/g lung or 10 mg/kg body weight), we estimate that the amount of surfactant present in the lungs of a normal 400 g guinea pig is about 4 mg. As the total amount of the aerosolized Surfactant TA inhaled in this study is about 1 - 2 mg, and its deposition to the lungs and pulmonary bronchi was approximately 46 %, therefore, the amount of the deposition of Surfactant TA to the lungs and pulmonary bronchi was considered to be about 1/8 - 1/4 of the amount normally present in the lung. The deposition of inhaled aerosol particles in the inspirator is due to the mechanisms of inertial impaction, Brownian diffusion, and gravitational settling, and the bronchial and alveolar deposition fraction can be estimated by its particle size. In obstruent disease, the fraction of deposition by impaction will increase. Since the mean particle size of PSF solution was about 3.6 μm and there was no difference in the particle size among PSF, saline and albumin solutions, we can estimate that the deposition sites of these solutions will be equal and they will mainly deposit on the upper airway after the bronchoconstriction.

In the present experiment, the administration of an aerosolized exogenous PSF, i.e., Surfactant TA, restored, to some extent, the abnormal dynamic compliance (Cdyn) and the pulmonary resistance (RL) that accompanied endogenous SRS-A mediated bronchoconstriction. At the lower dose, PSF was effective only on the recovery of Cdyn, but at the higher dose, it was effective in the recoveries of both Cdyn and RL. As Cdyn and RL are recognized as parameters responding to lower airway and upper airway, respectively, the results suggest that inhaled PSF is more effective on peripheral airways than on upper airways. On the other hand, pretreatment with inhaled PSF did not influence the injected histamine-induced bronchoconstriction which shows that PSF has neither a direct bronchodilative effect nor a nonspecific bronchoconstrictive property.

On the other hand, if surface tension is higher in the airway closest to the alveolous, it can be considered too that the liquid in the alveoli moves to the airways at the beginning of exhalation. The liquid will narrow the air channel, causing more liquid to move into the airways so that collapse of the alveoli occurs. Administration of PSF prevents this collapse.

From the above results, it can he said that administering an exogenous PSF (Surfactant TA) is effective against allergic bronchoconstriction.

Surfactant TA contains 1 % of proteins mainly consisting of hydrophobic apoproteins. These proteins are important from the point of view of structure, surface activity and surface film formation, and therefore it is desirable that PSF contains proteins. However, a synthetic PSF which lacks apoprotein, such as Exosurf, has the activity although weak.

Test results on ALEC, Exosurf, Alveofact, Surfactant CK and Humansurf obtained in the same manner as Surfactant TA are shown in Figs. 7-21.

[Effects on asthma patients]

Respiratory function tests were performed in terms of forced vital capacity (FVC), forced expiratory volume in one second (FEV1.0), maximum mid expiratory flow (MMF), $\Delta N_2$, total lung capacity (TLC), residual volume (RV), $PaO_2$ (arterial blood oxygen tension) and $PaCO_2$ (arterial blood carbon dioxide tension) (Rinshokensa Gijutsu Zensho, Vol.9, Physiological function tests: 367-393, Igaku Shoin). As the measurement systems, a dry spirometer (FUDAC-60, Fukuda Co., Tokyo, Japan) and Blood Gas System 278 (Ciba Corning Diagnostics Co. Medfield, U.S.A.) were used.

FVC, FEV1.0 and MMF were calculated from the maximum flow volume curve obtained by making the patients inspire to the maximum and expire as rapidly as possible. The slopes of alveolar plateau expressing $\Delta N_2$ was obtained from the nitrogen concentration-volume curves by single-breath nitrogen washout test. TLC were obtained by helium dilution closed circuit equibration method. The RV/TLC value means the residual volume rate. The study was completed within 90 minutes and the tests were performed before and 20 minutes after administration of PSF.

Eleven allergic asthma patients having asthmatic attacks were divided into a PSF group (n=6) and a control group (n=5). The PSF group inhaled, with 100 % $O_2$, 10 mg of Surfactant TA suspended in 1 ml physiological saline by a jet nebulizer (Type 95-B nebulizer system, Hitachi Ltd., Tokyo, Japan). The nebulizer operated by 51 l/min jet flow obtained from 3.5 $kgf/cm^2$ compressed air.

The results of measurements are shown in Tables 1 and 2.

Table 1

| Respiratory function tests | | | | | | | |
|---|---|---|---|---|---|---|---|
| Patient | | FCV (b) | FCV (a) | FEV 1.0 (b) | FEV 1.0 (a) | MMF (b) | MMF (a) |
| Group treated with PSF | 1 | 3.74 | 4.31 | 1.67 | 2.13 | 0.61 | 0.83 |
| | 2 | 2.13 | 2.56 | 1.06 | 1.42 | 0.48 | 0.67 |
| | 3 | 2.40 | 2.71 | 0.86 | 1.23 | 0.30 | 0.48 |
| | 4 | 1.71 | 2.02 | 0.65 | 0.71 | 0.26 | 0.25 |
| | 5 | 2.12 | 2.66 | 1.06 | 1.30 | 0.47 | 0.50 |
| | 6 | 1.63 | 1.91 | 1.02 | 1.25 | 0.45 | 0.69 |
| Control group | 7 | 2.99 | 2.93 | 1.71 | 1.68 | 0.78 | 0.82 |
| | 8 | 2.13 | 2.31 | 1.66 | 1.66 | 1.42 | 1.07 |
| | 9 | 1.32 | 1.98 | 0.66 | 0.67 | 0.37 | 0.22 |
| | 10 | 1.07 | 1.02 | 0.62 | 0.58 | 0.24 | 0.21 |
| | 11 | 2.52 | 2.45 | 1.41 | 1.38 | 0.47 | 0.45 |
| Patient | | RV (b) | RV (a) | TLC (b) | TLC (a) | $\Delta N_2$ (b) | $\Delta N_2$ (a) |
| Group treated with PSF | 1 | 184.47 | 139.13 | 131.89 | 121.14 | 0.64 | 0.57 |
| | 2 | 227.42 | 210.48 | 127.45 | 130.55 | 2.86 | 2.41 |
| | 3 | 249.12 | 180.12 | 112.22 | 105.34 | 7.12 | 4.19 |
| | 4 | 162.80 | 141.06 | 103.80 | 88.73 | 1.93 | 1.45 |
| | 5 | 208.28 | 146.15 | 120.39 | 107.98 | 1.61 | 1.39 |
| | 6 | 91.91 | 73.53 | 84.49 | 78.88 | 2.16 | 1.38 |
| Control group | 7 | 165.57 | 167.21 | 130.47 | 129.95 | 1.28 | 1.88 |
| | 8 | 71.67 | 74.17 | 78.99 | 79.79 | 1.19 | 2.39 |
| | 9 | 147.59 | 185.56 | 89.39 | 104.13 | 1.22 | 2.56 |
| | 10 | - | - | - | - | 6.42 | 8.08 |
| | 11 | 212.30 | 232.40 | 130.42 | 134.50 | 3.51 | 3.78 |

Table 2

| Blood gas test | | | | | | | |
|---|---|---|---|---|---|---|---|
| Patient | | pH(b) | pH(a) | $PaCO_2$ (b) | $PaCO_2$ (a) | $PaO_2$ (b) | $PaO_2$ (a) |
| Group treated with PSF | 1 | 7.430 | 7.45 | 42.5 | 37.6 | 53.0 | 64.3 |
| | 2 | 7.382 | 7.376 | 39.1 | 35.6 | 72.1 | 72.9 |
| | 3 | 7.440 | 7.434 | 38.0 | 37.8 | 68.7 | 72.6 |
| | 4 | 7.430 | 7.421 | 44.5 | 43.8 | 74.4 | 98.2 |
| | 5 | 7.449 | 7.423 | 32.6 | 36.5 | 68.0 | 72.0 |
| | 6 | 7.404 | 7.424 | 36.0 | 33.1 | 67.1 | 77.2 |

Table 2 (continued)

| Blood gas test | | | | | | | |
|---|---|---|---|---|---|---|---|
| Patient | | pH(b) | pH(a) | $PaCO_2$ (b) | $PaCO_2$ (a) | $PaO_2$ (b) | $PaO_2$ (a) |
| Control group | 7 | 7.405 | 7.409 | 33.5 | 33.1 | 73.2 | 70.5 |
| | 8 | 7.425 | 7.420 | 32.7 | 30.1 | 74.7 | 73.2 |
| | 9 | 7.510 | 7.500 | 39.3 | 39.6 | 55.7 | 54.1 |
| | 10 | 7.337 | 7.368 | 50.8 | 48.5 | 50.2 | 52.7 |
| | 11 | 7.360 | 7.356 | 39.1 | 39.8 | 72.3 | 70.5 |

In the respiratory function tests and blood gas test, the effect of administering PSF is expressed as a percentage according to the following formula and the results are shown in Table 3.

$$\frac{[RF(after\ PSF) - RF(before\ PSF)]}{RF\ (before\ PSF)} \times 100$$

(RF means respiratory function data)

Table 3

| | FVC | FEV1.0 | MMF |
|---|---|---|---|
| PSF group | 18.2 ± 1.2 | 26.5 ± 4.7 | 31.9 ± 10.4 |
| control group | 9.7 ± 10.3 | -1.8 ± 1.3 | -15.4 ± 8.0 |
| | RV | TLC | RV/TLC |
| PSF group | -20.5 ± 3.5 | -7.2 ± 2.3 | -14.2 ± 3.7 |
| control group | 9.9 ± 5.6 | -5.1 ± 3.9 | 3.5 ± 1.5 |
| | $\Delta N_2$ | $PaCO_2$ | $PaO_2$ |
| PSF group | -23.7 ± 5.1 | -3.1 ± 3.5 | 13.5 ± 4.8 |
| control group | 58.2 ± 20.3 | -2.2 ± 1.8 | -1.2 ± 1.6 |

In the control group, FVC, FEV1.0, MMF and $PaO_2$ were almost unchanged although $\Delta N_2$ increased significantly. In the PSF-administered group, FVC, FEV1.0, MMF, $PaO_2$ and $\Delta N_2$ were markedly improved in all patients.
Moreover, the means of TLC and RV had no significant change in the control group, but were significantly decreased after inhalation in the PSF group. The RV/TLC was also decreased in the PSF group.
This means that PSF exhibits the effect of bronchodilation on constricting bronchioles by stabilizing the bronchioles, causing maximum ventilation on the peripheral alveoli and preventing air trapping, and as a result inhibits asthmatic symptoms.
Blood gas analysis test revealed no significant difference in $PaCO_2$ between both groups. In addition, $PaO_2$ was significantly increased after treatment in the PSF group but had no change in the control group. This suggests that PSF improves incorporation of oxygen into the alveoli.
In all patients of the PSF group, asthmatic attacks and symptoms were relieved. On the other hand, in 5 patients of the control group, these were not changed or worsened.
As is stated, it can be concluded that the drug containing PSF as effective ingredient is a useful antasthmatic.

[Acute toxity test]

To male ICR mice aged 5 weeks and male Wister rats aged 5 weeks, Surfactant TA was administered by oral and intraperitoneal route to obtain $LD_{5\emptyset}$s. In the mice, the $LD_{5\emptyset}$ was 3 g/kg or more by oral route and 2g/kg or more by intraperitoneal route. Similarly, in the rats, $LD_{5\emptyset}$ was 4 g/kg or more by oral route and 2.5 g/kg or more by intraperitoneal route.

[Subacute toxicity test]

To mature Wister rats, Surfactant TA was intraperitoneally administered at a dose of 500 mg/kg for one month. There were no changes in body weight nor findings by gross and histological observations of the lungs or the other main organs in the rats one month later. Moreover, no biological abnormalities caused by heterologous protein were found.

[Method of use and dosage]

The antiasthmatic medicament provided by the use of the present invention contains 1 - 50 mg of PSF as one dose for an adult. As to use, the above-mentioned dose is suspended in an electrolyte solution such as water or physiological saline to obtain a concentration of 0.1 - 100 mg/ml, and injected or sprayed into the air way before and after onset of asthma. As the frequency of administration, 1 - 10 times are adequate. Depending on patients' symptoms or combined treatments, the above-mentioned dosage, method of use and frequency may be changed suitably.
As necessary, the antasthmatic medicament may contain pharmaceutical additives including stabilizer, preservative, isotonizing agents, buffers or suspensions, or bactericides. As dosage form, liquid or powder used by suspending on need is appropriate. This drug is packed in an airtight container such as a vial and an ampule, and preserved as an aseptic preparation.

## Claims

1. The use of a pulmonary surface active material in the manufacture of a medicament for the treatment of asthma in humans.

2. Use as claimed in claim 1, wherein the pulmonary surface active material contains 40% or more of phospholipids.

3. Use as claimed in claim 2, wherein a main component of the phospholipids is phosphatidyl choline or choline phosphoglyceride.

4. Use as claimed in any one of the preceding claims, wherein the pulmonary surface active material is one selected from the group consisting of:

    (i) surface active material containing phospholipids, neutral lipids, total cholesterol, free fatty acids, carbohydrates and proteins, originating from mammalian pulmonary tissue, at respectively 68.6-90.7 w/w %, 0.3-13.0 w/w %, 0.0-8.0 w/w %, 1.0-27.7 w/w %, 0.1-2.0 w/w % and 0.0-3.5 w/w % of the total dry weight;

    (ii) a synthetic pulmonary surface active material containing 80-95 w/w % of phosphatidyl choline, cardiolipin and/or phosphatidyl glycerol, as phospholipids, 5-20 w/w % of neutral lipids and 0-10 w/w % of fatty acids;

    (iii) a surfactant comprising 50.6-85.0 w/w % choline phosphoglyceride, 4.5-37.6 w/w % acid phospholipids, 4.6-24.6 w/w % fatty acids, and 0.1-10.0 w/w % mammalian lung-derived lipoproteins;

    (iv) a synthetic surfactant comprising 53.9-87.8 w/w % choline phosphoglyceride, 4.8-38.2 w/w % acid phospholipids, and 7.0-26.2 w/w % of fatty acids;

    (v) a material consisting of lipids extracted from porcine alveolar washing solution to which calcium chloride has been added;

    (vi) a natural pulmonary surface active material obtained from bovine alveoli and comprising phopholipids, cholesterols, hydrophobic surface active proteins, free fatty acids, triglycerides and calcium (trade name ALVEOFACT).

    (vii) a natural pulmonary surface active material obtained from human waters (trade name HUMANSURF);

    (viii) a synthetic pulmonary surface active material comprising Surfactant CK, dipalmitoyl phosphatidyl choline, hexadecanol, tyloxapol and sodium chloride (trade name EXOSURF);

(ix) a synthetic pulmonary surface active material comprising 7 parts of dipalmitoyl phosphatidyl choline and 3 parts of phosphatidyl glycerol (trade name ALEC).

**5.** Use as claimed in any one of the preceding claims, wherein said medicament is an aerosol preparation having an average particle size of about 3.6 μm.

**6.** Use as claimed in claim 5, wherein said aerosol preparation is able to dose said pulmonary surfactant in an amount of 1 to 50 mg per application.

**7.** Use as claimed in any one of claims 5 and 6, wherein said pulmonary surfactant is Surfactant TA.

**Patentansprüche**

**1.** Verwendung eines lungenoberflächenaktiven Materials zur Herstellung eines Medikamentes für die Behandlung von Asthma beim Menschen.

**2.** Verwendung nach Anspruch 1, wobei das lungenoberflächenaktive Material 40 % oder mehr Phospholipide enthält.

**3.** Verwendung nach Anspruch 2, wobei eine wesentliche Komponente der Phospholipide Phosphatidylcholin oder Cholinphosphoglycerid ist.

**4.** Verwendung nach einem der Ansprüche 1 bis 3, wobei das lungenoberflächenaktive Material aus der Gruppe bestehend aus

(i) einem oberflächenaktive Material, welches aus Lungengewebe von Säugetieren gewonnen ist, enthaltend Phospholipide, neutrale Lipide, Gesamt-Cholesterin, freie Fettsäuren, Kohlehydrate und Proteine, mit einem Anteil an der Gesamttrockenmasse von jeweils 68,6 - 90,7 Gew.-%, 0,3 - 13,0 Gew.-%, 0,0 - 8,0 Gew.-%, 1,0 - 27,7 Gew.-%, 0,1 - 2,0 Gew.-% und 0,0 bis 3,5 Gew.-%;
(ii) einem synthetischen lungenoberflächenaktiven Material enthaltend 80 - 95 Gew.-% Phosphatidylcholin, Cardiolipin und/oder Phosphatidylglycerin als Phospholipide, 5 - 20 Gew.-% neutrale Lipide und 0 - 10 Gew.-% Fettsäuren;
(iii) einem oberflächenaktiven Stoff enthaltend 50,6 - 85,0 Gew.-% Cholinphosphoglycerid, 4,5 - 37,6 Gew.-% saure Phospholipide, 4,6 - 24,6 Gew.-% Fettsäuren und 0,1 - 10,0 Gew.-% aus der Lunge von Säugetieren gewonnene Lipoproteine;
(iv) einem synthetischen oberflächenaktiven Stoff enthaltend 53,9 - 87,8 Gew.-% Cholinphosphoglycerid, 4,8 - 38,2 Gew.-% saure Phospholipide und 7,0 - 26,2 Gew.-% Fettsäuren;
(v) einem Material bestehend aus aus einer Waschlösung für Lungenbläschen von Schweinen extrahierten Lipiden, zu welchen Calciumchlorid gegeben wurde;
(vi) einem natürlichen lungenoberflächenaktiven Material, welches aus Lungenbläschen von Rindern gewonnen wurde und Phospholipide, Cholesterine, hydrophobe oberflächenaktive Proteine, freie Fettsäuren, Triglyceride und Calcium enthält (Handelsname ALVEOFACT);
(vii) einem natürlichen lungenoberflächenaktiven Material, welches aus menschlicher Körperflüssigkeit gewonnen wurde (Handelsname HUMANSURF);
(viii) einem synthetischen lungenoberflächenaktiven Material enthaltend Surfactant CK, Dipalmitoylphosphatidylcholin, Hexadekanol, Tyloxapol und Natriumchlorid (Handelsname EXOSURF);
(ix) einem synthetischen lungenoberflächenaktiven Material enthaltend 7 Teile Dipalmitoylphosphatidylcholin und 3 Teile Phosphatidylglycerin (Handelsname ALEC)
ausgewählt ist.

**5.** Verwendung nach einem der vorhergehenden Ansprüche, wobei das Medikament eine Aerosol-Formulierung mit einer durchschnittlichen Partikelgröße von etwa 3,6 μm ist.

**6.** Verwendung nach Anspruch 5, wobei die Aerosol-Formulierung in der Lage ist, das lungenoberflächenaktive Material in einer Menge von 1 bis 50 mg pro Applikation zu dosieren.

**7.** Verwendung nach einem der Ansprüche 5 oder 6, dadurch gekennzeichnet, daß das lungenoberflächenaktive Material Surfactant TA ist.

## Revendications

1. Utilisation d'une substance tensio-active pulmonaire dans la fabrication d'un médicament destiné au traitement de l'asthme chez les humains.

2. Utilisation telle que revendiquée dans la revendication 1, dans laquelle la substance tensio-active pulmonaire contient 40 % ou plus de phospholipides.

3. Utilisation telle que revendiquée dans la revendication 2, dans laquelle un constituant principal des phospholipides est une phosphatidylcholine ou un phosphoglycéride de choline.

4. Utilisation telle que revendiquée dans l'une quelconque des revendications précédentes, dans laquelle la substance tensio-active pulmonaire est choisie dans le groupe formé par :

   (i) une substance tensio-active contenant des phospholipides, des lipides neutres, du cholestérol total, des acides gras libres, des glucides et des protéines, provenant de tissu pulmonaire de mammifère, à des taux respectifs de 68,6 à 90,7 % en poids, 0,3 à 13,0 % en poids, 0,0 à 8,0 % en poids, 1,0 à 27,7 % en poids, 0,1 à 2,0 % en poids et 0,0 à 3,5 % en poids du poids sec total ;
   (ii) une substance tensio-active pulmonaire synthétique contenant 80 à 95 % en poids de phosphatidylcholine, de cardiolipine et/ou de phosphatidylglycérol, comme phospholipides, 5 à 20 % en poids de lipides neutres et 0 à 10 % en poids d'acides gras ;
   (iii) un agent tensio-actif comprenant 50,6 à 85,0 % en poids de phosphoglycéride de choline, 4,5 à 37,6 % en poids de phospholipides acides, 4,6 à 24,6 % en poids d'acides gras et 0,1 à 10,0 % en poids de lipoprotéines dérivées de poumon de mammifère ;
   (iv) un agent tensio-actif synthétique comprenant 53,9 à 87,8 % en poids de phosphoglycéride de choline, 4,8 à 38,2 % en poids de phospholipides acides et 7,0 à 26,2 % en poids d'acides gras ;
   (v) une substance consistant en lipides extraits d'une solution de lavage alvéolaire de porc à laquelle a été ajouté du chlorure de calcium ;
   (vi) une substance tensio-active pulmonaire naturelle obtenue à partir d'alvéoles bovins et comprenant des phospholipides, des cholestérols, des protéines tensio-actives hydrophobes, des acides gras libres, des triglycérides et du calcium (nom commercial ALVEOFACT) ;
   (vii) une substance tensio-active pulmonaire naturelle obtenue à partir de liquide amniotique humain (nom commercial HUMANSURF) ;
   (viii) une substance tensio-active pulmonaire synthétique comprenant du Surfactant CK, de la dipalmitoylphosphatidylcholine, de l'hexadécanol, du tyloxapol et du chlorure de sodium (nom commercial EXOSURF) ;
   (ix) une substance tensio-active pulmonaire synthétique comprenant 7 parties de dipalmitoylphosphatidylcholine et 3 parties de phosphatidylglycérol (nom commercial ALEC).

5. Utilisation telle que revendiquée dans l'une quelconque des revendications précédentes, dans laquelle ledit médicament est une préparation pour aérosol ayant une taille moyenne de particules d'environ 3,6 μm.

6. Utilisation telle que revendiquée dans la revendication 5, dans laquelle ladite préparation pour aérosol est capable de doser ladite substance tensio-active pulmonaire en une quantité de 1 à 50 mg par application.

7. Utilisation telle que revendiquée dans l'une quelconque des revendications 5 et 6, dans laquelle ladite substance tensio-active pulmonaire est Surfactant TA.

FIG.1

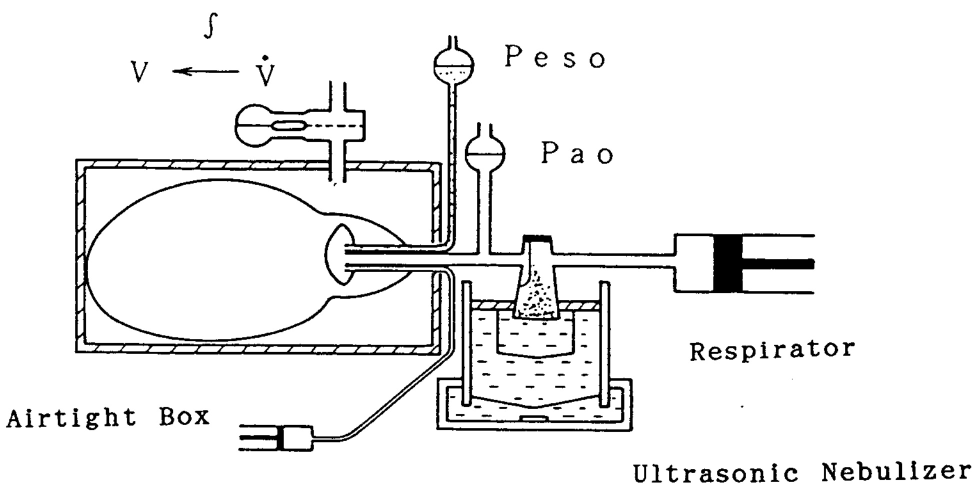
∫
V ← V̇
Peso
Pao
Respirator
Airtight Box
Ultrasonic Nebulizer

FIG.2

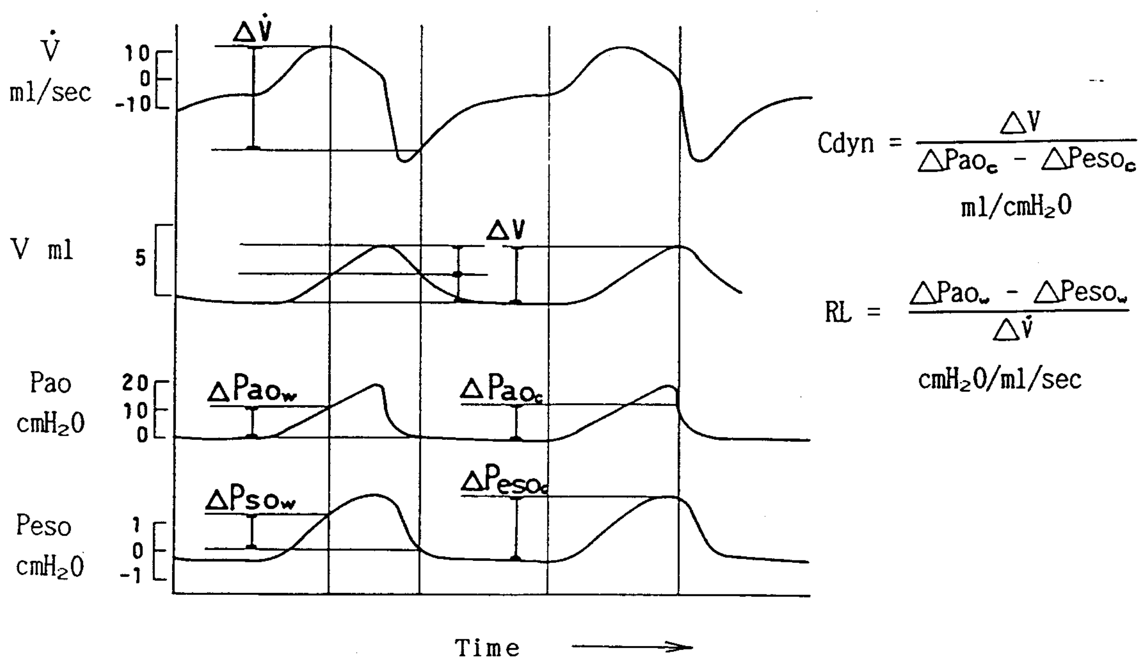
V̇
ml/sec
10
0
-10
ΔV̇
V ml
5
ΔV
Pao
cmH2O
20
10
0
ΔPaow
ΔPaoc
Peso
cmH2O
1
0
-1
ΔPsow
ΔPesoc
Time
Cdyn = ΔV / (ΔPaoc - ΔPesoc)
ml/cmH2O
RL = (ΔPaow - ΔPesow) / ΔV̇
cmH2O/ml/sec

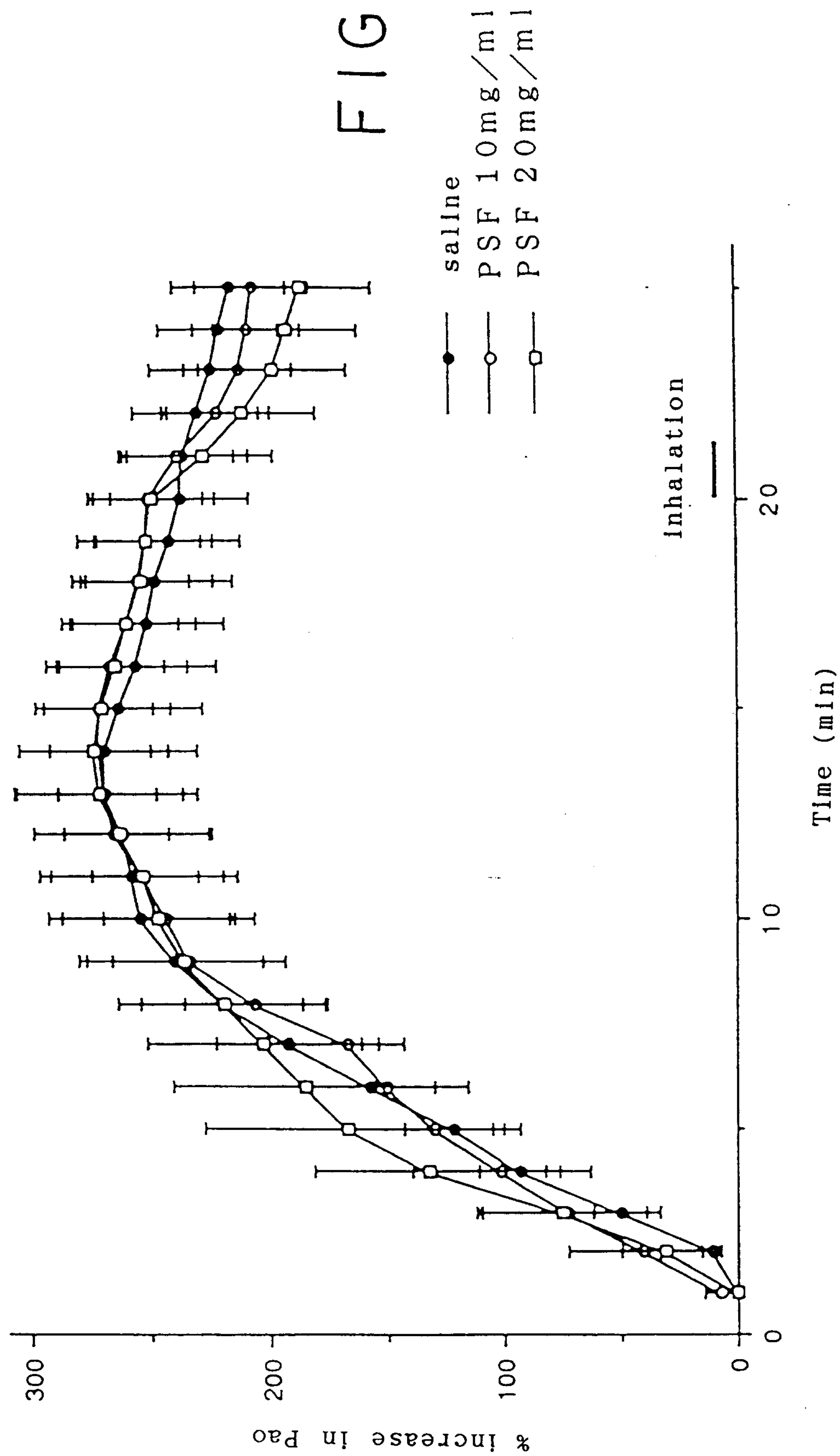
saline
PSF 10mg/ml
PSF 20mg/ml
% increase in Pao
300
200
100
0
0
10
20
Time (min)
Inhalation

FIG. 3

FIG. 4

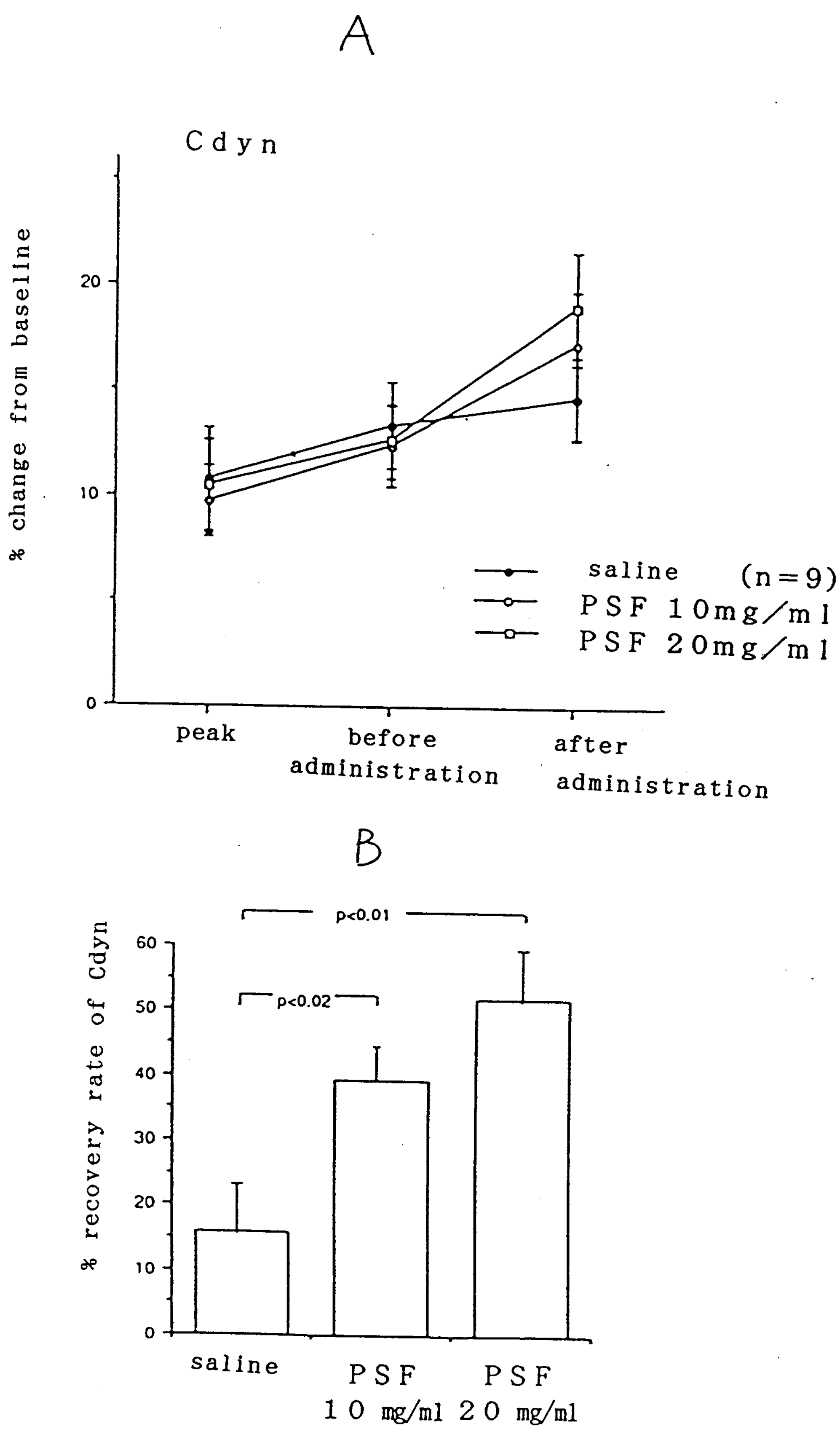
A
Cdyn
% change from baseline
20
10
0
saline (n=9)
PSF 10mg/ml
PSF 20mg/ml
peak
before administration
after administration
B
p<0.01
p<0.02
% recovery rate of Cdyn
60
50
40
30
20
10
0
saline
PSF 10 mg/ml
PSF 20 mg/ml

FIG.5

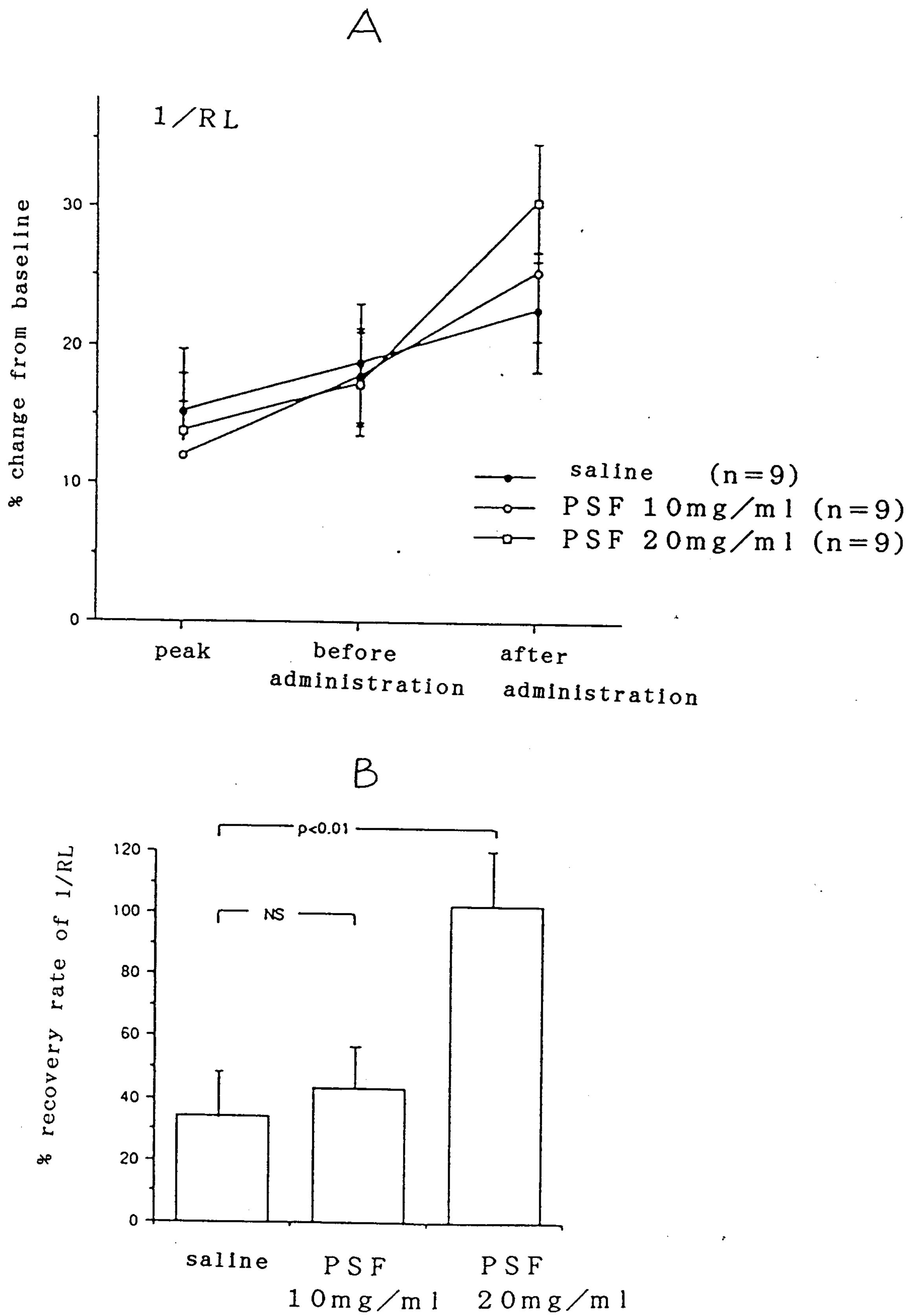
A
1/RL
% change from baseline
0
10
20
30
peak
before administration
after administration
saline (n=9)
PSF 10mg/ml (n=9)
PSF 20mg/ml (n=9)
B
p<0.01
NS
% recovery rate of 1/RL
0
20
40
60
80
100
120
saline
PSF 10mg/ml
PSF 20mg/ml

FIG.6

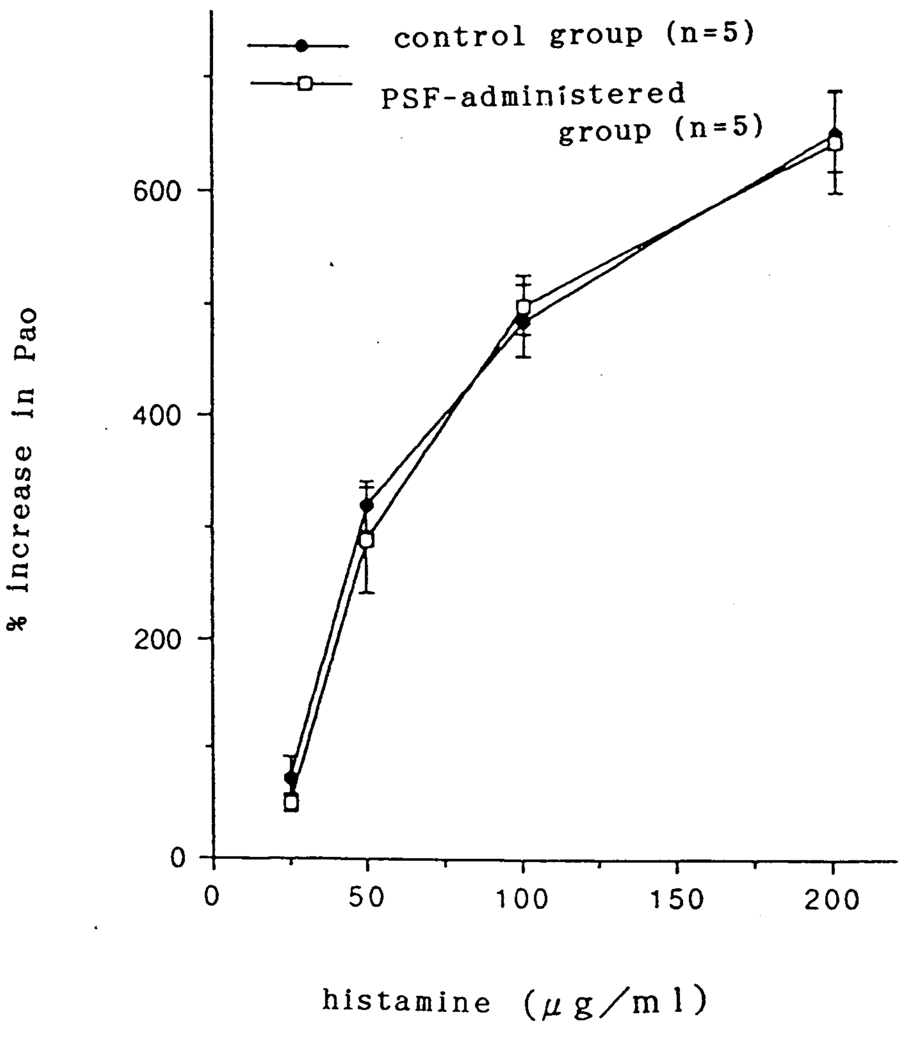
control group (n=5)
PSF-administered group (n=5)
% increase in Pao
0
200
400
600
0
50
100
150
200
histamine (μg/ml)

FIG.7

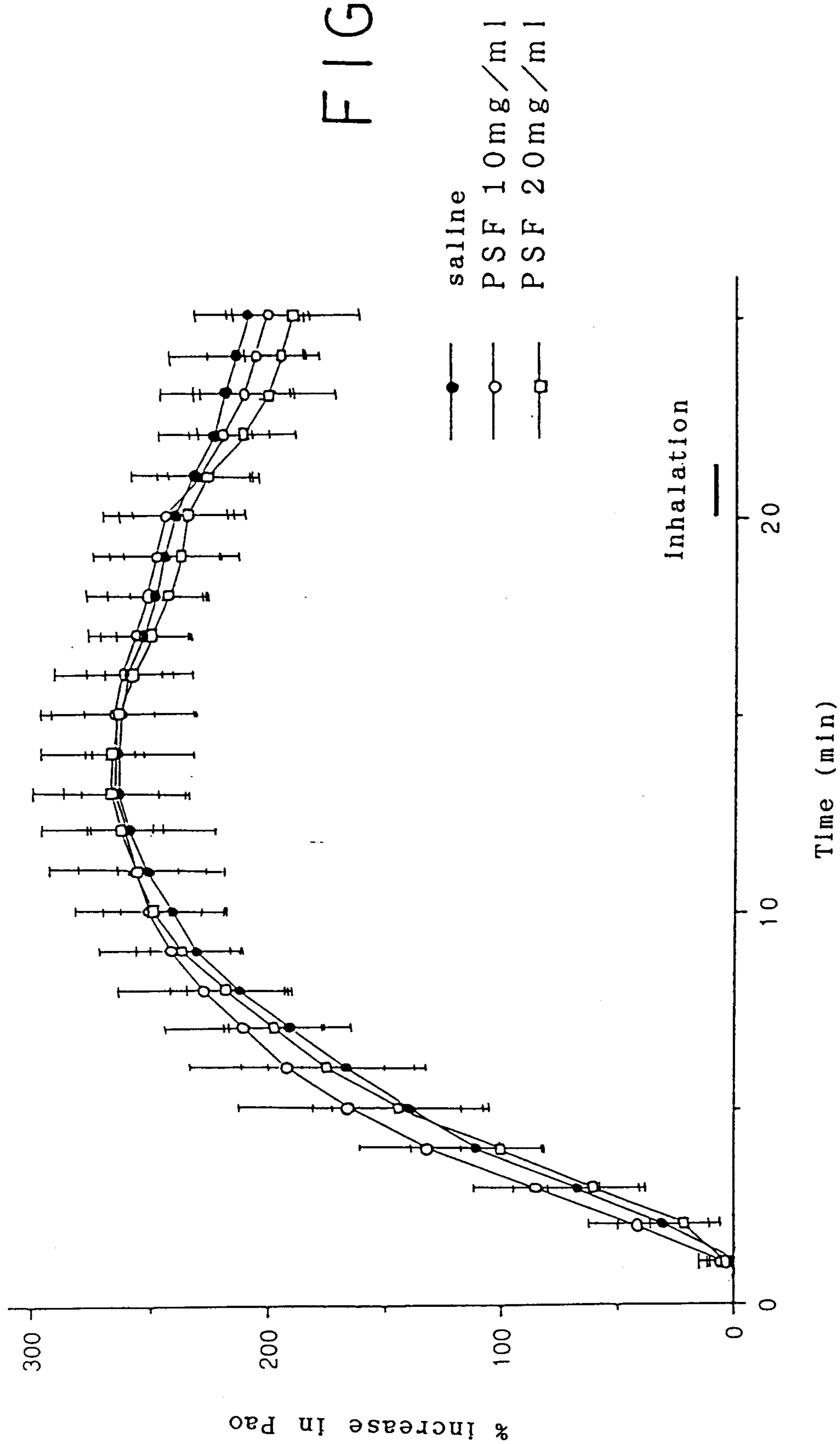
saline
PSF 10mg/ml
PSF 20mg/ml
Inhalation
% increase in Pao
300
200
100
0
0
10
20
Time (min)

FIG.8

A

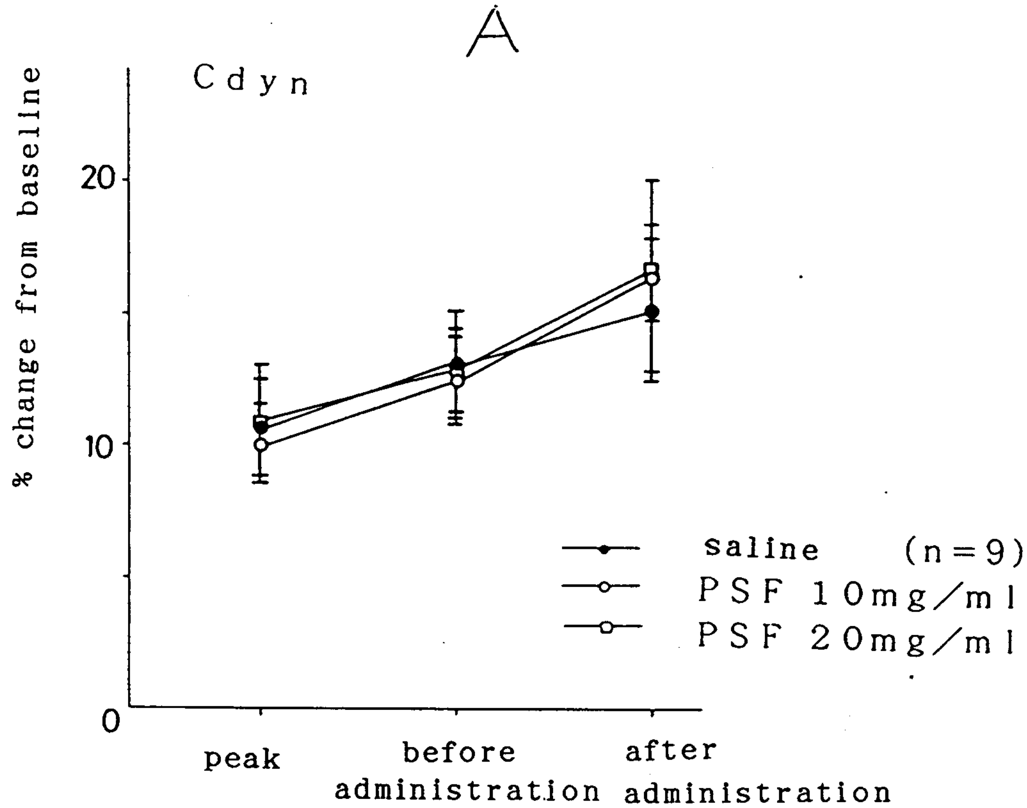
Cdyn
% change from baseline
20
10
0
saline (n=9)
PSF 10mg/ml
PSF 20mg/ml
peak
before administration
after administration

B

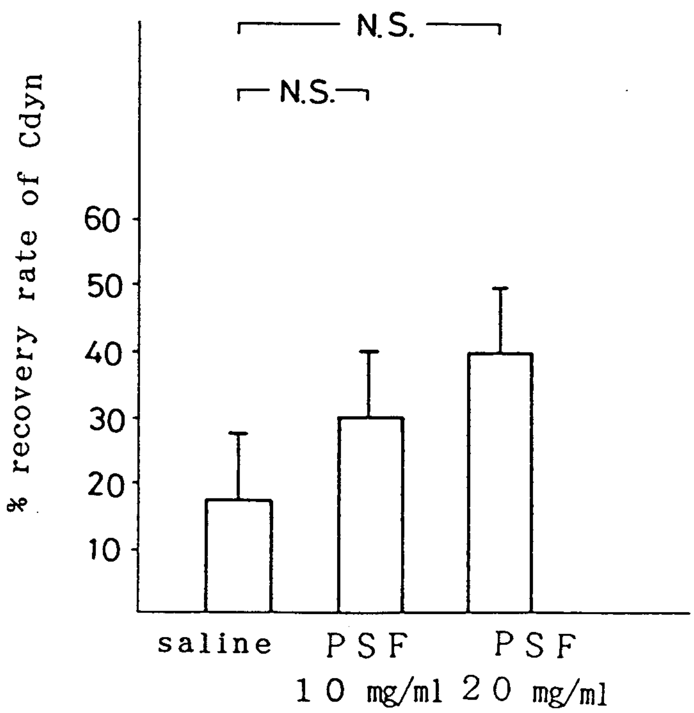
N.S.
N.S.
% recovery rate of Cdyn
60
50
40
30
20
10
saline
PSF 10 mg/ml
PSF 20 mg/ml

FIG. 9

A

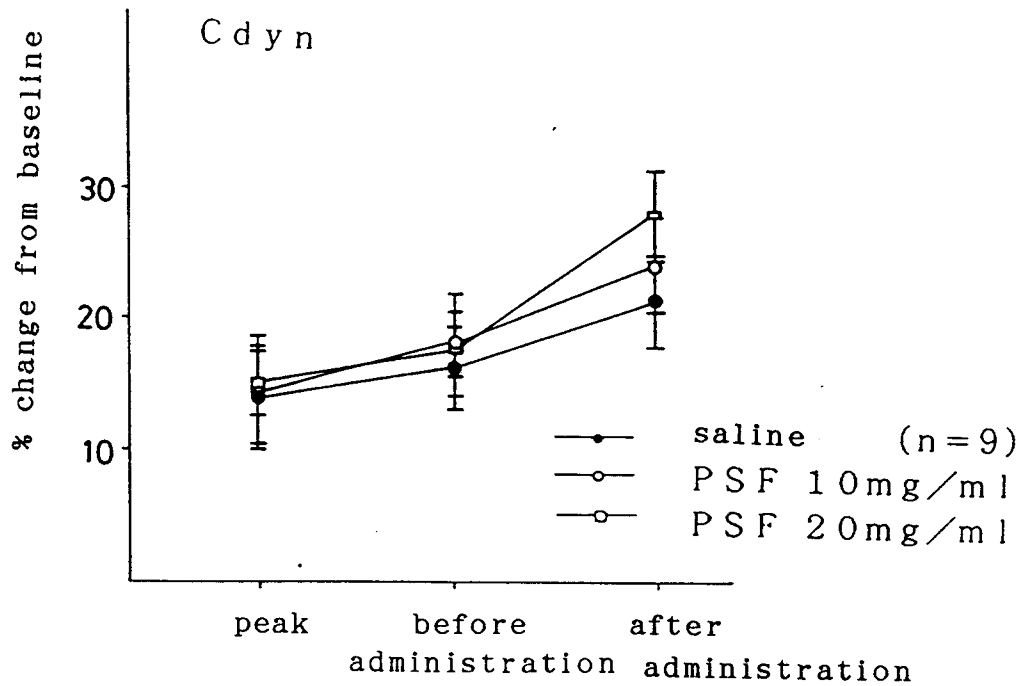
Cdyn
% change from baseline
30
20
10
saline (n=9)
PSF 10mg/ml
PSF 20mg/ml
peak
before administration
after administration

B

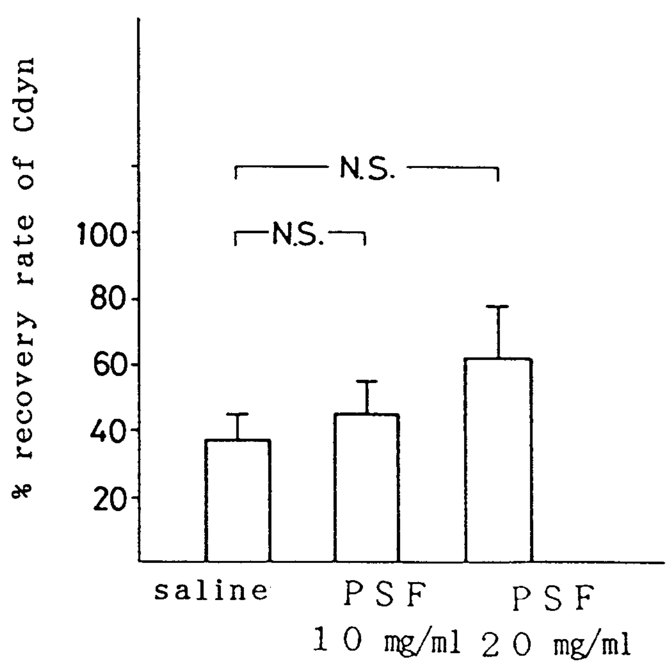
% recovery rate of Cdyn
100
80
60
40
20
N.S.
N.S.
saline
PSF 10 mg/ml
PSF 20 mg/ml

FIG.10

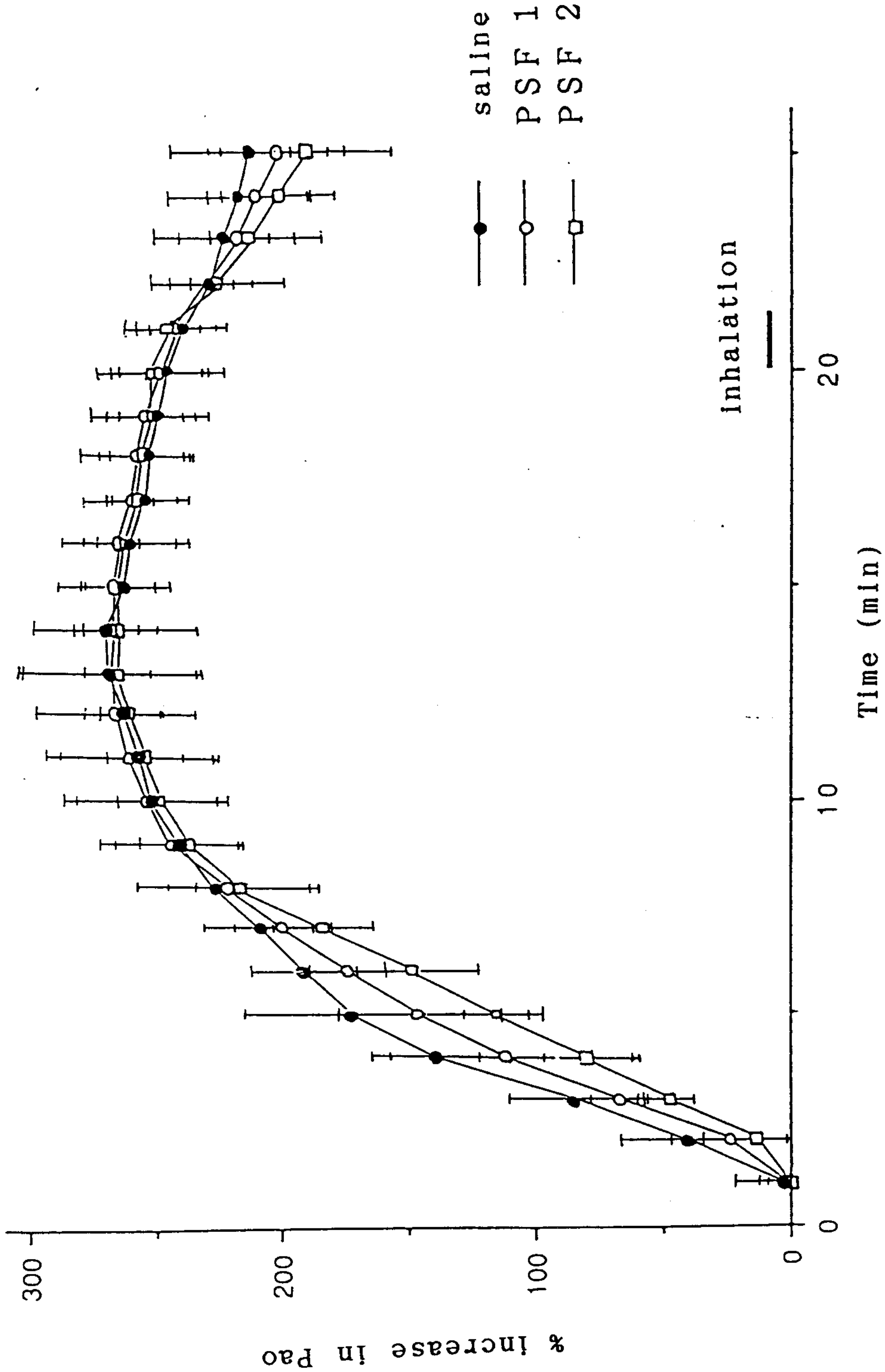
saline
PSF 10mg/ml
PSF 20mg/ml
Inhalation
% increase in Pao
300
200
100
0
Time (min)
0
10
20

FIG.11

A

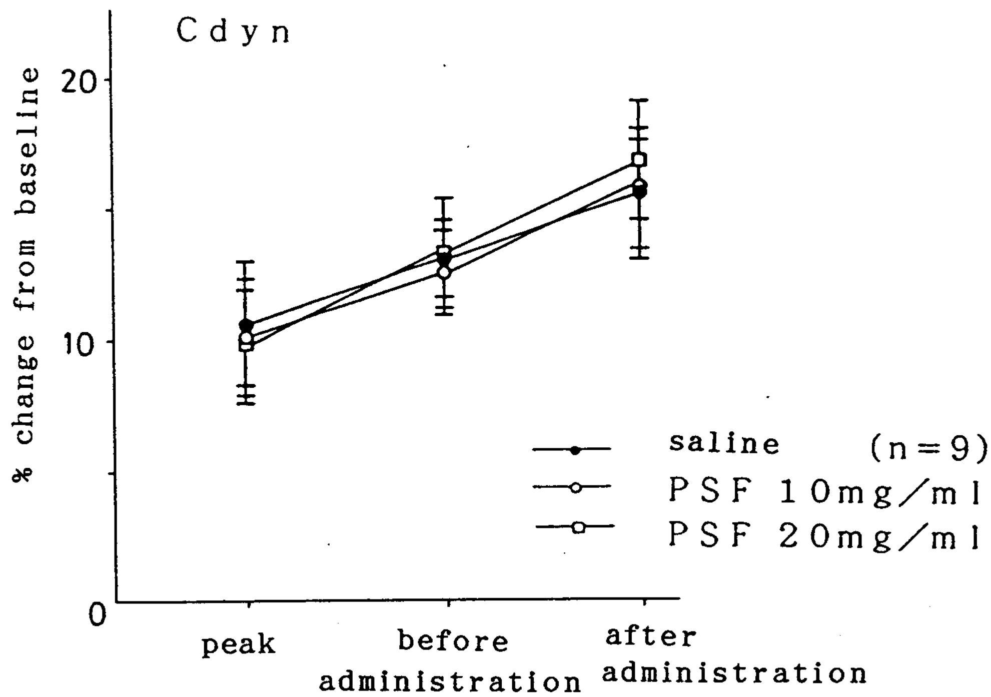
Cdyn
% change from baseline
20
10
0
peak
before administration
after administration
saline (n=9)
PSF 10mg/ml
PSF 20mg/ml

B

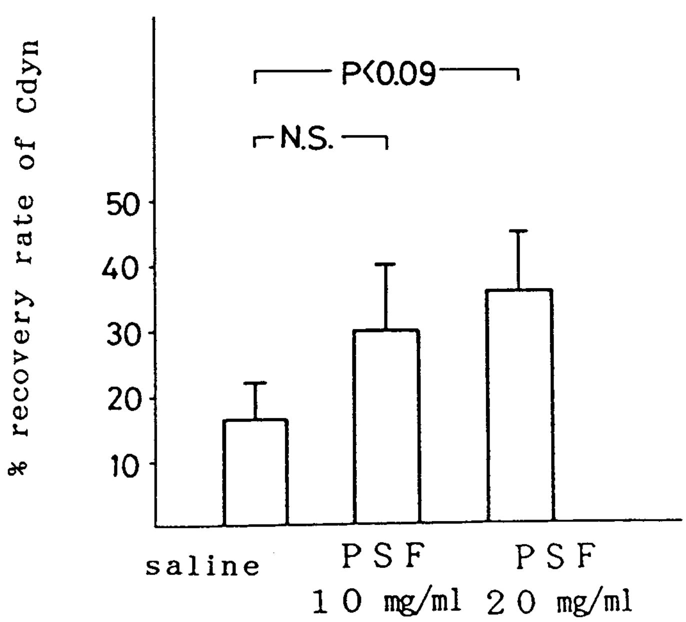
% recovery rate of Cdyn
P<0.09
N.S.
50
40
30
20
10
saline
PSF 10 mg/ml
PSF 20 mg/ml

FIG.12

A

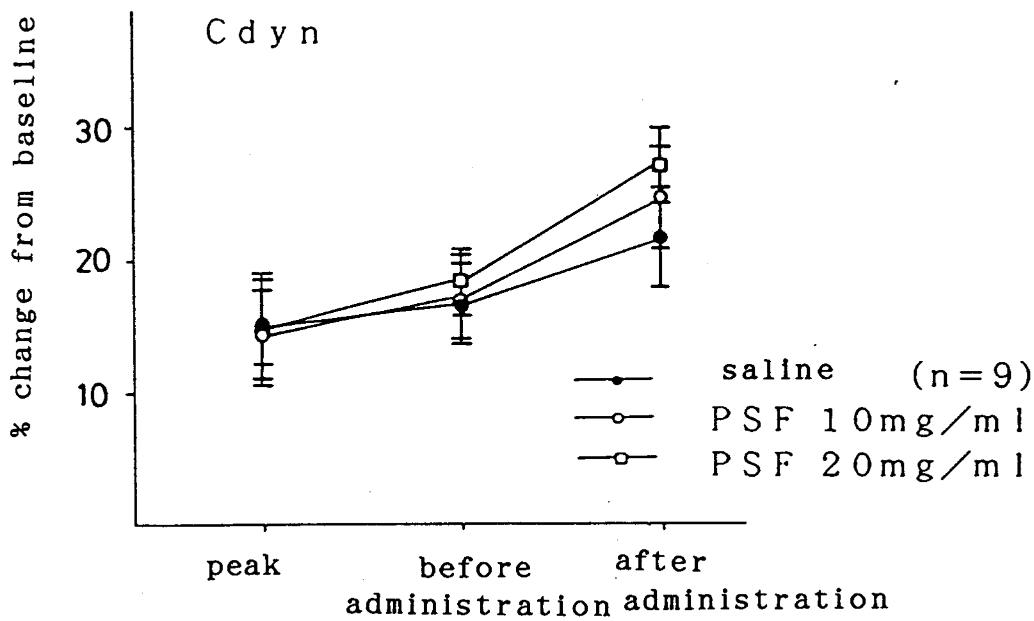
Cdyn
% change from baseline
30
20
10
saline (n=9)
PSF 10mg/ml
PSF 20mg/ml
peak
before administration
after administration

B

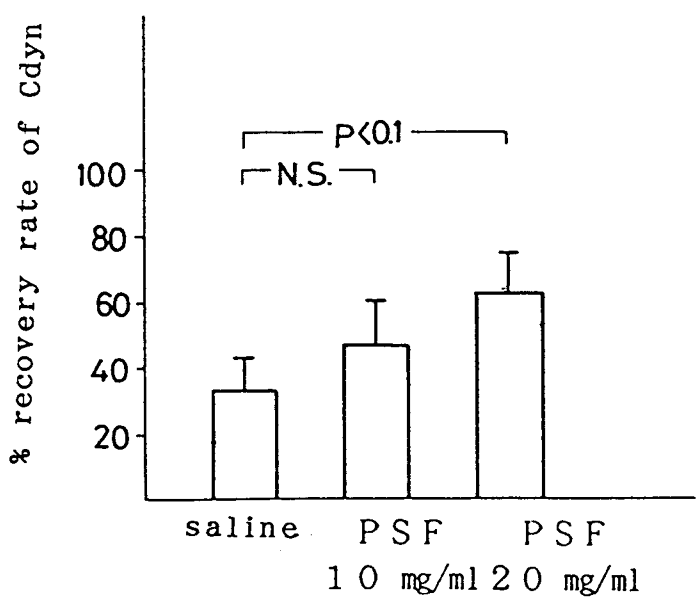
% recovery rate of Cdyn
100
80
60
40
20
P<0.1
N.S.
saline
PSF 10 mg/ml
PSF 20 mg/ml

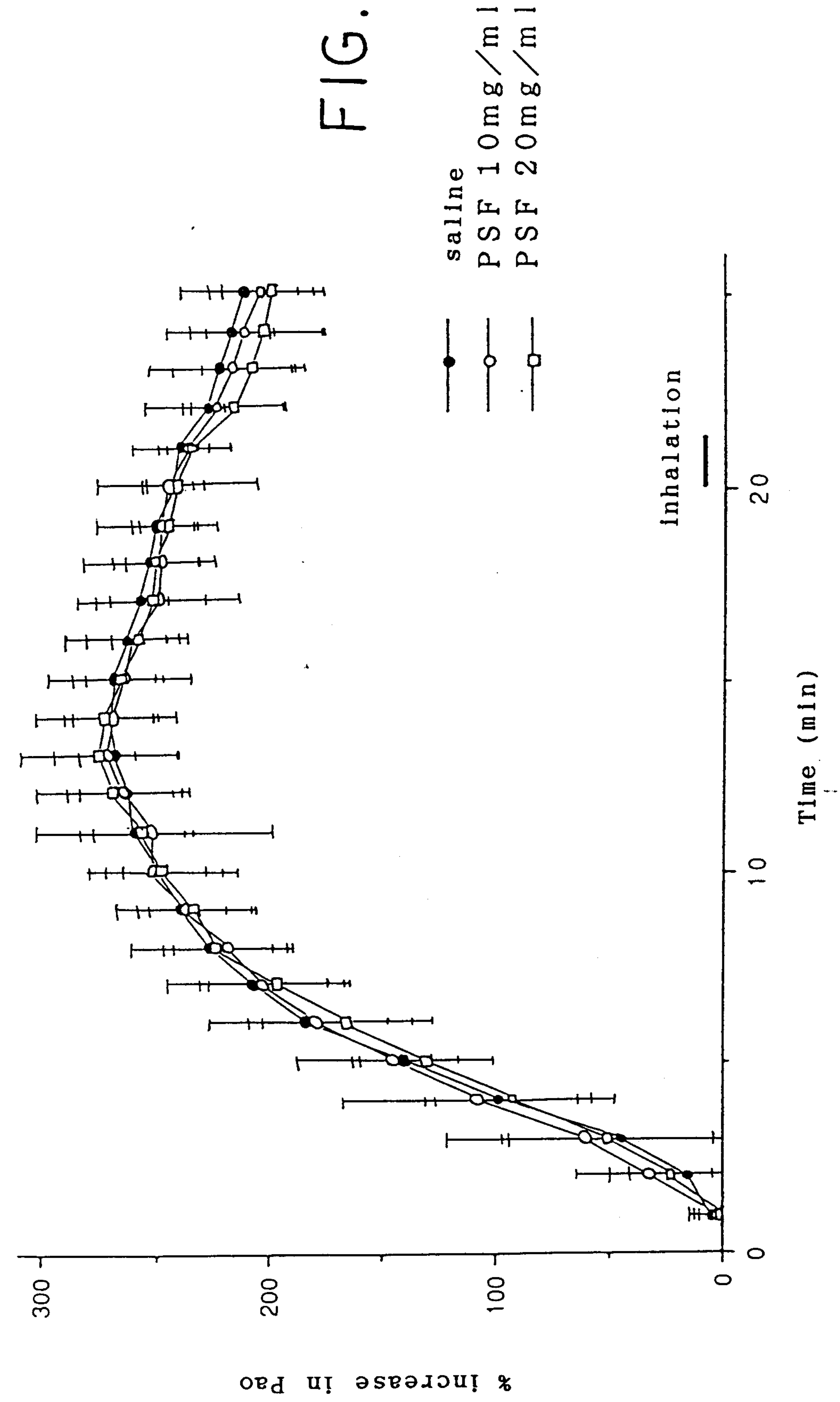

saline
PSF 10mg/ml
PSF 20mg/ml
inhalation
Time (min)
% increase in Pao
0
10
20
0
100
200
300


FIG.13

FIG.14

A

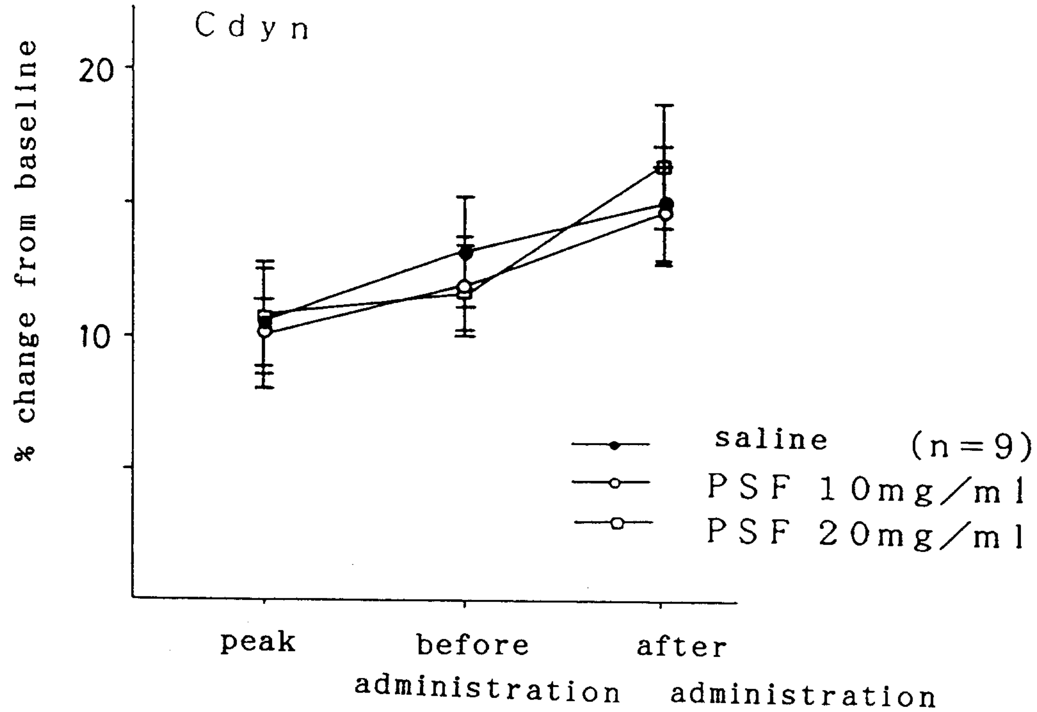
Cdyn
% change from baseline
20
10
saline (n=9)
PSF 10mg/ml
PSF 20mg/ml
peak
before administration
after administration

B

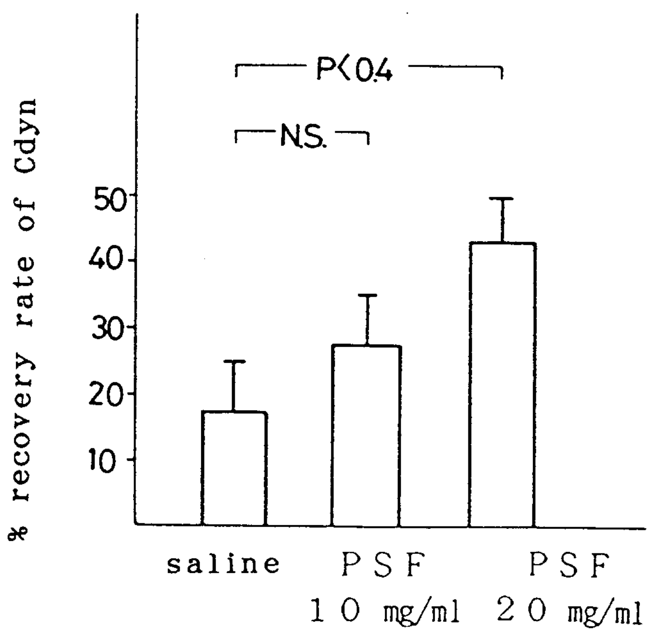
P<0.4
N.S.
% recovery rate of Cdyn
50
40
30
20
10
saline
PSF 10 mg/ml
PSF 20 mg/ml

FIG.15

A

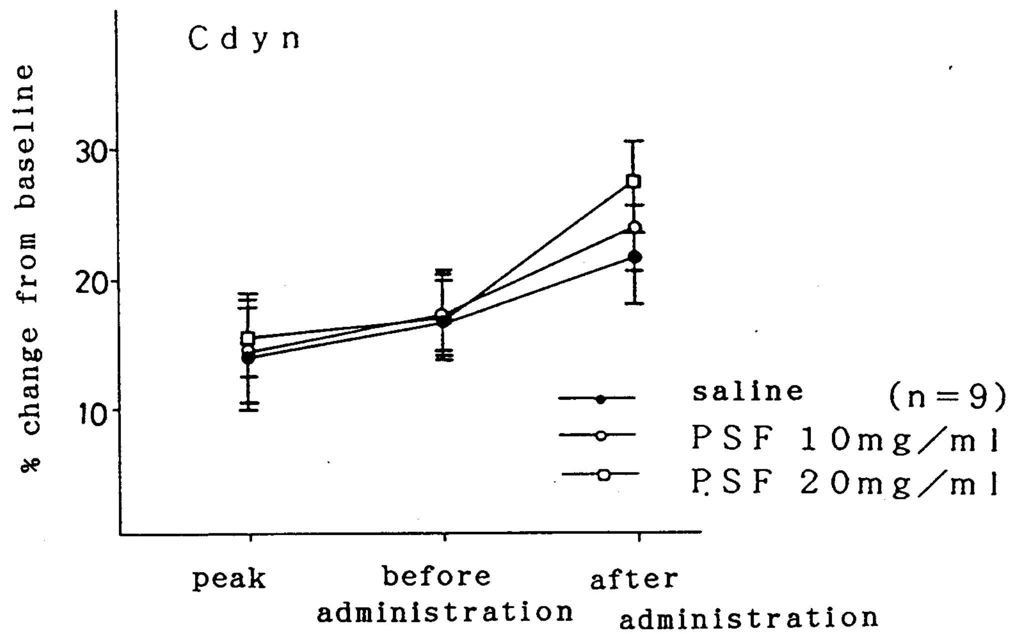
Cdyn
% change from baseline
30
20
10
saline (n=9)
PSF 10mg/ml
PSF 20mg/ml
peak
before administration
after administration

B

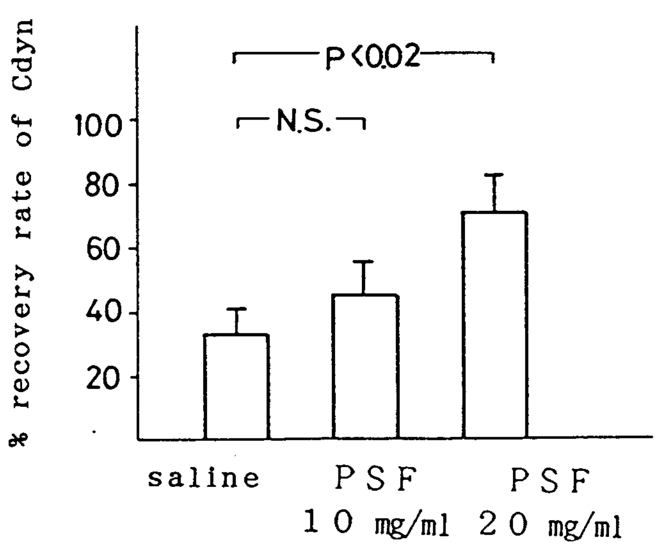
% recovery rate of Cdyn
P<0.02
N.S.
100
80
60
40
20
saline
PSF 10 mg/ml
PSF 20 mg/ml

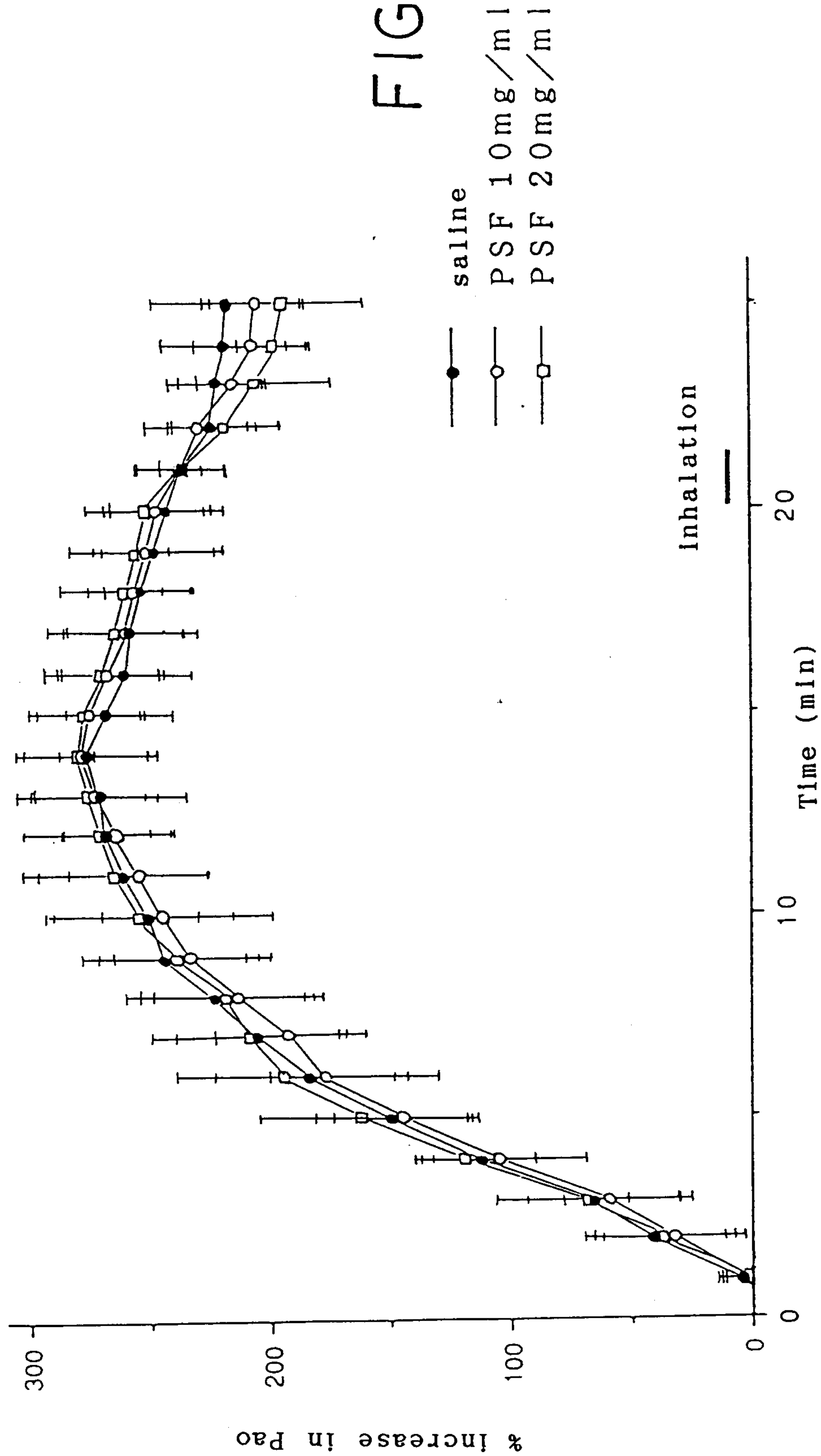
saline
PSF 10mg/ml
PSF 20mg/ml
Inhalation
Time (min)
% increase in Pao
0
10
20
0
100
200
300

FIG.16

FIG.17

A

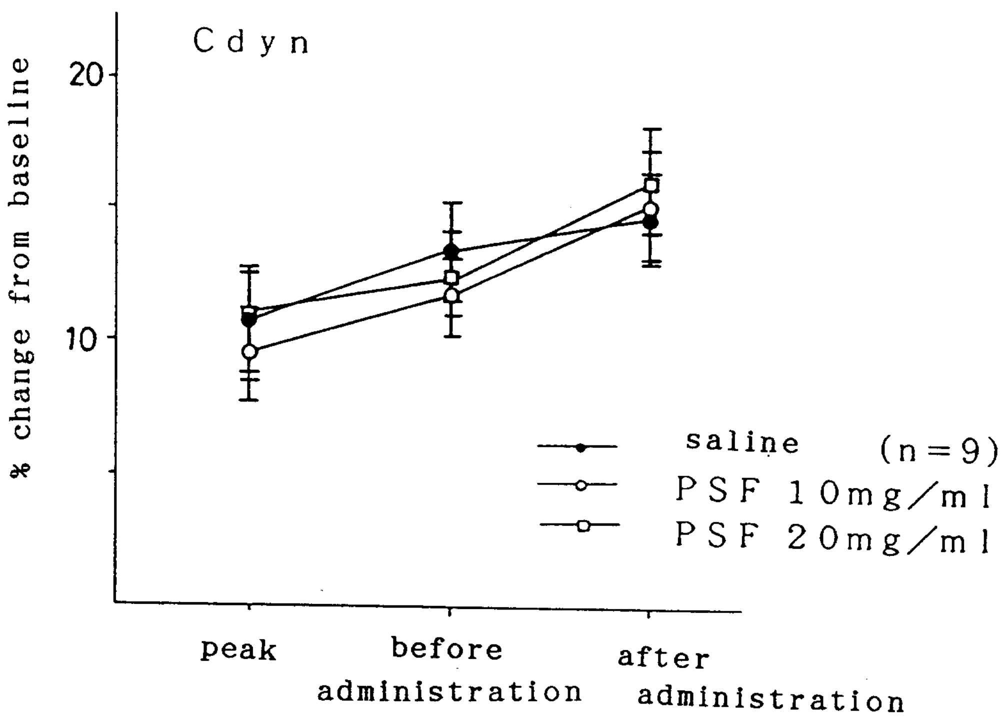
Cdyn
20
10
% change from baseline
saline (n=9)
PSF 10mg/ml
PSF 20mg/ml
peak
before administration
after administration

B

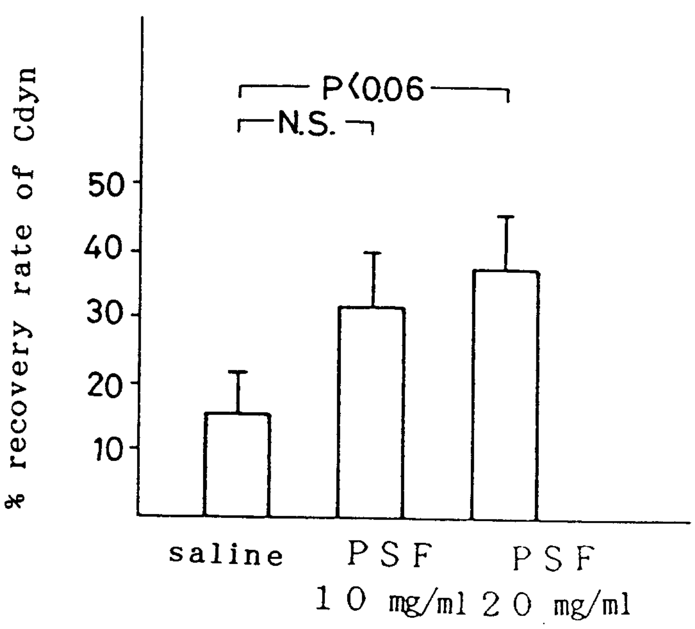
P<0.06
N.S.
50
40
30
20
10
% recovery rate of Cdyn
saline
PSF 10 mg/ml
PSF 20 mg/ml

FIG.18

A

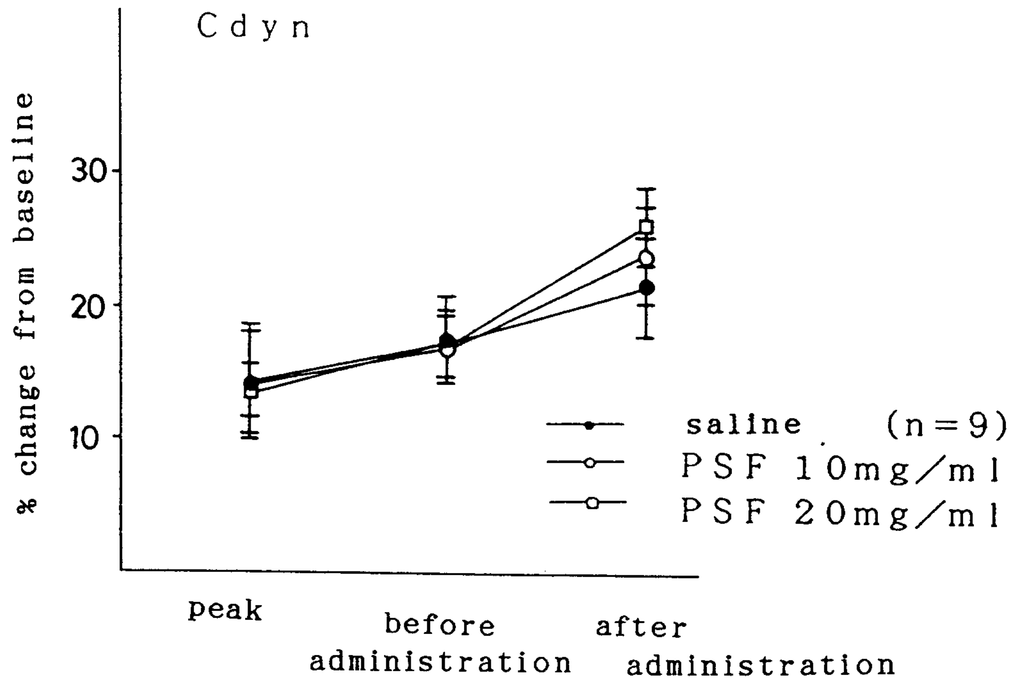
Cdyn
% change from baseline
30
20
10
saline (n=9)
PSF 10mg/ml
PSF 20mg/ml
peak
before administration
after administration

B

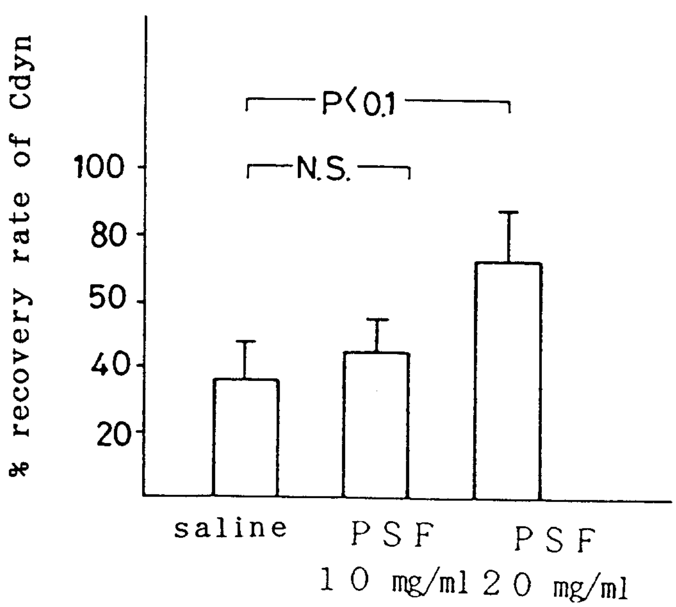
% recovery rate of Cdyn
P<0.1
N.S.
100
80
50
40
20
saline
PSF 10 mg/ml
PSF 20 mg/ml

FIG.19

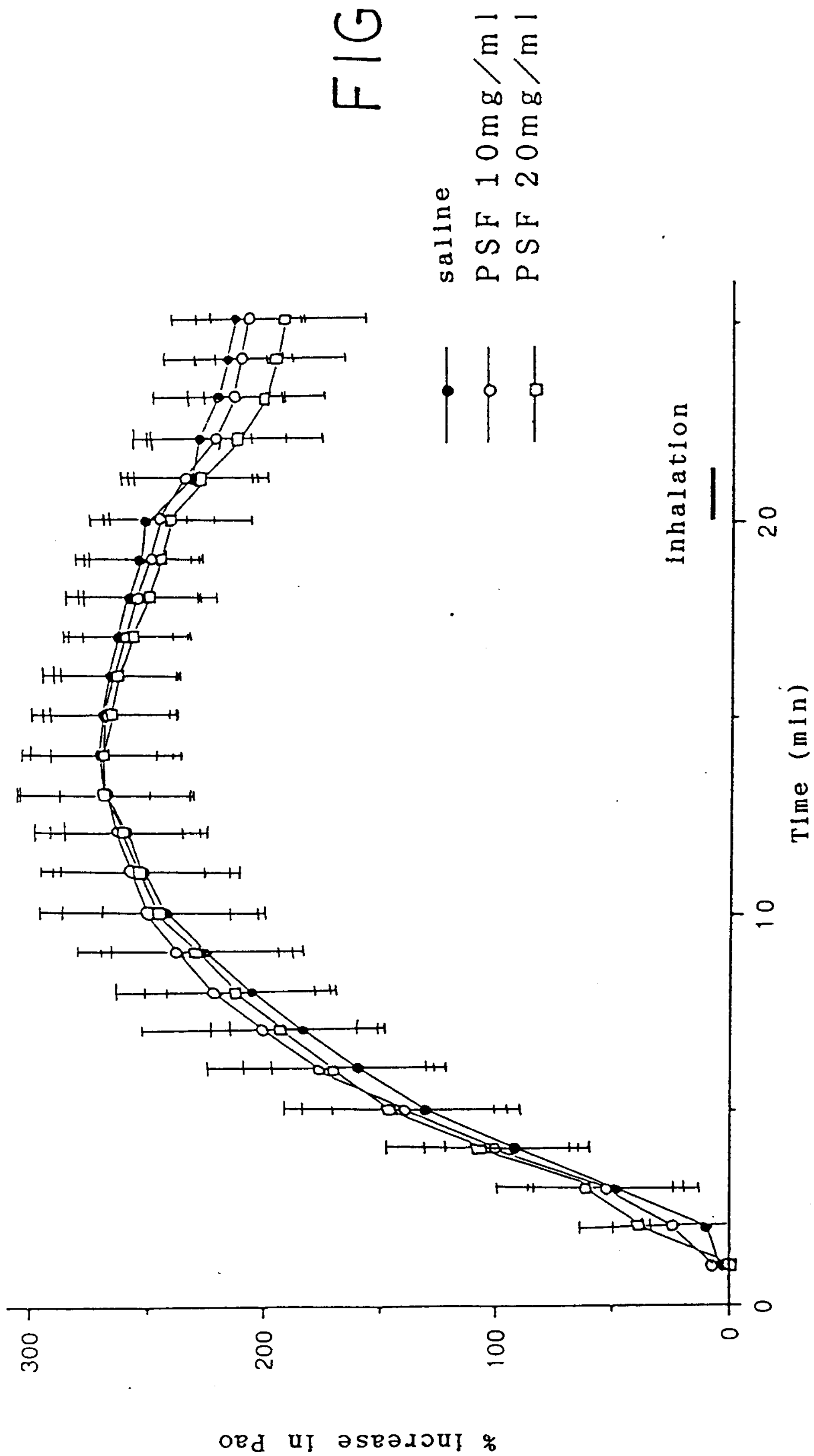
saline
PSF 10mg/ml
PSF 20mg/ml
% increase in Pao
300
200
100
0
0
10
20
Time (min)
Inhalation

FIG.20

A

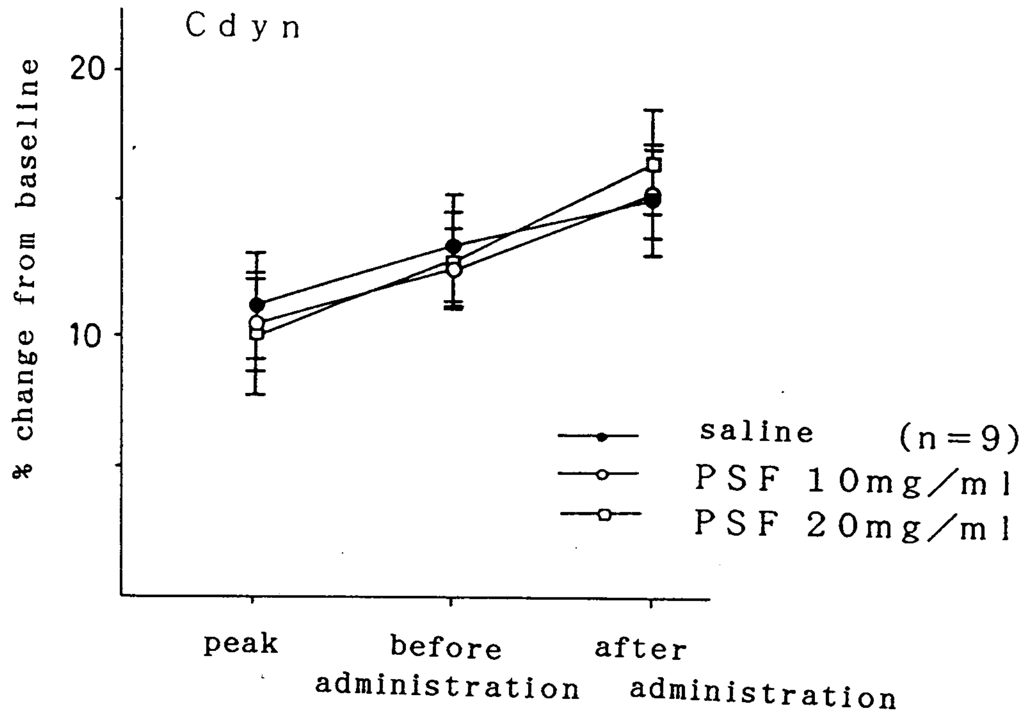
Cdyn
% change from baseline
20
10
saline (n=9)
PSF 10mg/ml
PSF 20mg/ml
peak
before administration
after administration

B

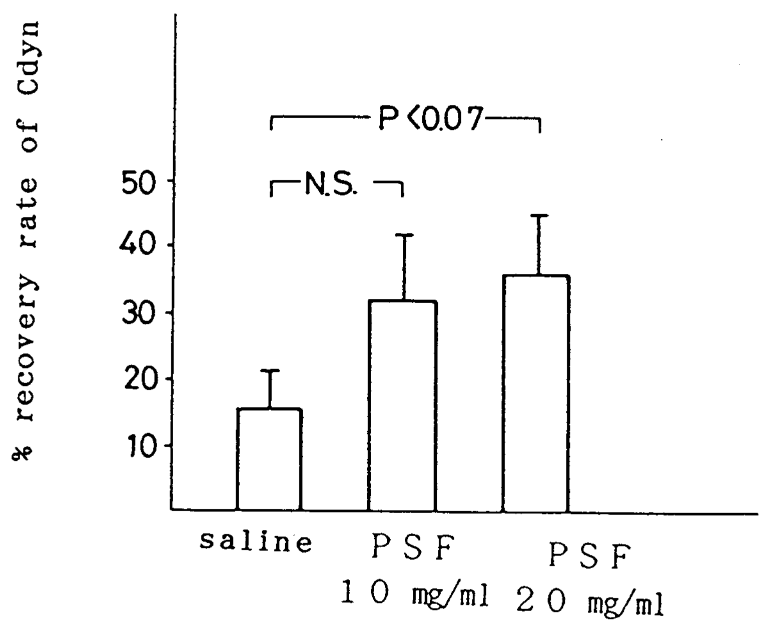
% recovery rate of Cdyn
P<0.07
N.S.
50
40
30
20
10
saline
PSF 10 mg/ml
PSF 20 mg/ml

FIG.21

A

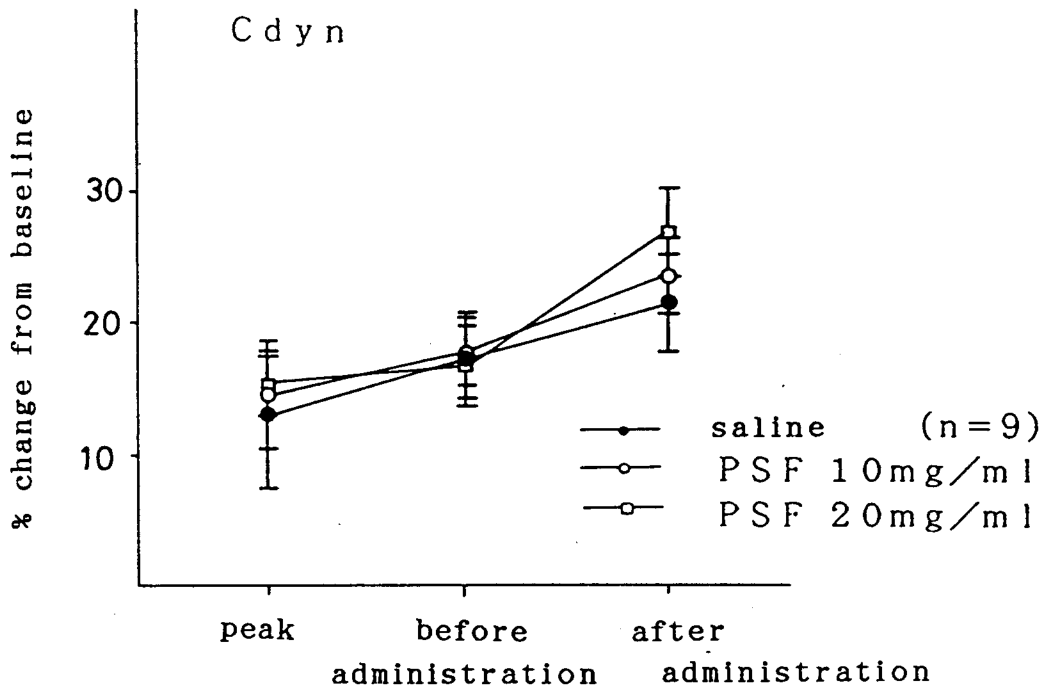
Cdyn
% change from baseline
30
20
10
saline (n=9)
PSF 10mg/ml
PSF 20mg/ml
peak
before administration
after administration

B

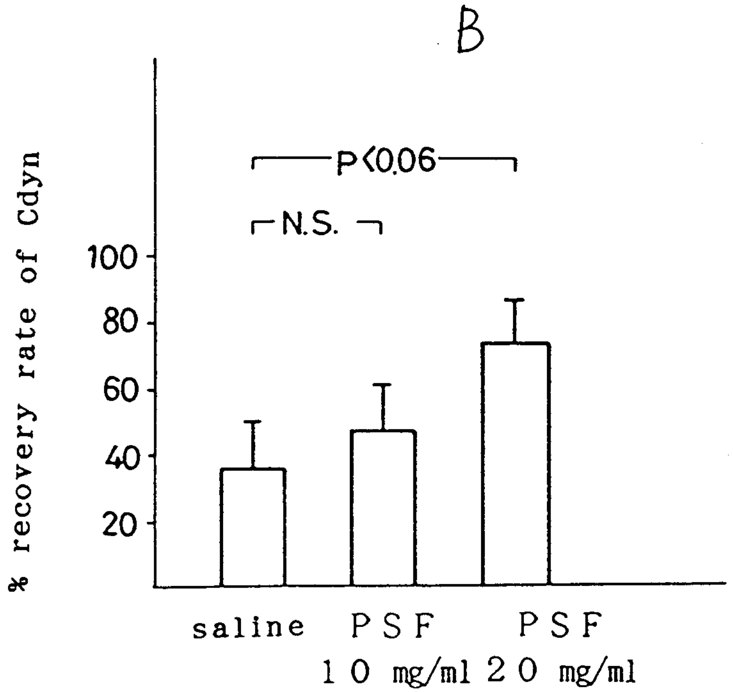
P<0.06
N.S.
% recovery rate of Cdyn
100
80
60
40
20
saline
PSF 10 mg/ml
PSF 20 mg/ml